(19) Europäisches Patentamt
European Patent Office
Office européen des brevets

(11) Veröffentlichungsnummer: **0 126 986**
**B1**

(12) # EUROPÄISCHE PATENTSCHRIFT

(45) Veröffentlichungstag der Patentschrift: **05.08.87**

(51) Int. Cl.[4]: **C 07 K 1/06**, C 07 K 5/06, C 07 C 143/68

(21) Anmeldenummer: **84104690.7**

(22) Anmeldetag: **26.04.84**

(54) **Verfahren zur Herstellung von N-alkylierten Dipeptiden und deren Estern.**

(30) Priorität: **28.04.83 DE 3315464**

(43) Veröffentlichungstag der Anmeldung: **05.12.84 Patentblatt 84/49**

(45) Bekanntmachung des Hinweises auf die Patenterteilung: **05.08.87 Patentblatt 87/32**

(84) Benannte Vertragsstaaten: **AT BE CH DE FR GB IT LI LU NL SE**

(56) Entgegenhaltungen:
**EP - A - 0 046 953**

**ANGEWANDTE CHEMIE, Band 95, Heft 1, Januar 1983, F. EFFENBERGER et al. "Trifluormethansulfonate von alpha-Hydroxycarbonsäureestern - Edukte zur racemisierungsfreien Synthese N-substituierter alpha-Aminosäuren", Seite 50**
**Perspectives in Peptide Chemistry, pp. 15-30 (Karger, Basel 1981)**

(73) Patentinhaber: **HOECHST AKTIENGESELLSCHAFT, Postfach 80 03 20, D-6230 Frankfurt am Main 80 (DE)**

(72) Erfinder: **Henning, Rainer, Dr., Rotenhofstrasse 31, D-6234 Hattersheim am Main (DE)**
Erfinder: **Urbach, Hansjörg, Dr., Le Lavandoustrasse 41, D-6242 Kronberg/Taunus (DE)**

**EP 0 126 986 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Verbindungen der Formel I

```
        *           *       *
R3OOC-CH-N - C -CH-NH-CH-(CH2)n-R          (I)
         |   |  ||  |       |
        R4  R5  O   R1      COOR2
```

in welcher

n 1 oder 2 ist,

R Wasserstoff,
einen gegebenenfalls substituierten aliphatischen Rest mit 1 bis 8 C-Atomen,
einen gegebenenfalls substituierten cycloaliphatischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 14 C-Atomen,
einen gegebenenfalls substituierten cycloaliphatisch-aliphatischen Rest mit 7 - 14 C-Atomen,
einen Rest $OR^a$ oder $SR^a$, worin

$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,

$R^1$ Wasserstoff,
einen gegebenenfalls substituierten aliphatischen Rest mit 1 bis 6 C-Atomen,
einen gegebenenfalls substituierten cycloaliphatischen Rest mit 3 - 9 C-Atomen,
einen gegebenenfalls substituierten cycloaliphatisch-aliphatischen Rest mit 4 - 13 C-Atomen,
einen gegebenenfalls substituierten aromatischen Rest mit 6 - 12 C-Atomen,
einen gegebenenfalls substituierten araliphatischen Rest mit 7 - 16 C-Atomen,
einen gegebenenfalls substituierten heteroaromatischen Rest mit 5 - 12 Ringatomen oder
die Seitenkette einer gegebenenfalls geschützten natürlich vorkommenden α-Aminosäure bedeuten,

$R^2$ und $R^3$ gleich oder verschieden sind und Wasserstoff, einen gegebenenfalls substituierten aliphatischen Rest mit 1 - 6 C-Atomen,
einen gegebenenfalls substituierten cycloaliphatischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 16 C-Atomen bedeuten und

$R^4$ und $R^5$ zusammen mit den sie tragenden Atomen ein heterocyclisches mono-, bi-, oder tricyclisches Ringsystem mit 3 bis 15 C-Atomen bilden.

Als solche Ringsysteme kommen insbesondere solche aus der folgenden Gruppe in Betracht:

Pyrrolidin (A); Piperidin (B); Tetrahydroisochinolin (C); Decahydroisochinolin (D); Octahydroindol (E); Octahydrocyclopenta[b]pyrrol (F); 2-Aza-bicyclo[2.2.2]octan (G); 2-Azabicyclo[2.2.1]heptan (H); 2-Azaspiro[4.5]decan (I); 2-Azaspiro[4.4]nonan (J); Spiro[(bicyclo[2.2.1]heptan)-2,3-pyrrolidin] (K); Spiro[(bicyclo[2.2.2]octan)-2,3-pyrrolidin] (L); 2-Azatricyclo[4.3.0.1$^{6,9}$]decan (M); Decahydrocyclohepta[b]pyrrol (N); Octahydroisoindol (O); Octahydrocyclopenta[c]pyrrol (P); 2,3,3a,4,5,7a-Hexahydroindol (Q); Tetrahydrothiazol (R); die alle gegebenenfalls substituiert sein können. Bevorzugt sind jedoch die unsubstituierten Systeme.

Die in Betracht kommenden cyclischen Aminosäureester weisen die folgenden Strukturformeln auf.

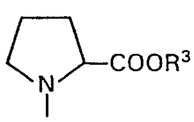

A

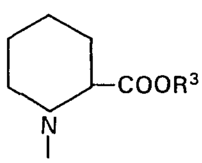

B

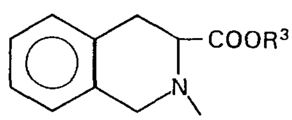

C

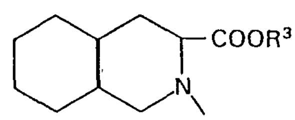

D

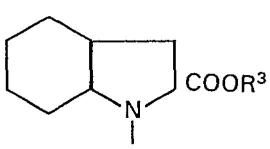

E

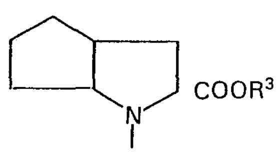

F

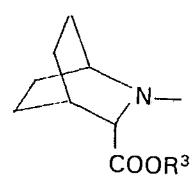

G

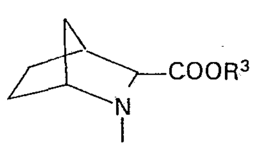

H

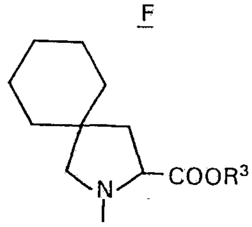

I

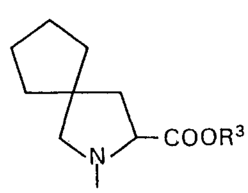

J

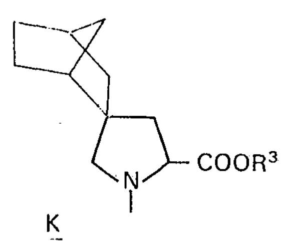

K

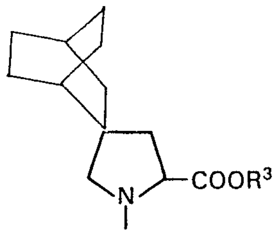

L

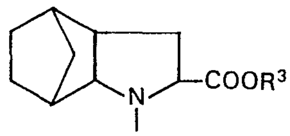

M

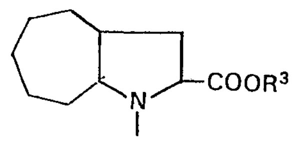

N

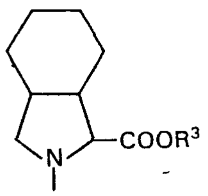

O

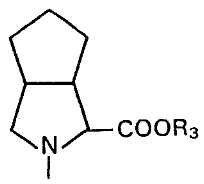

P

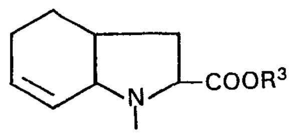

Q

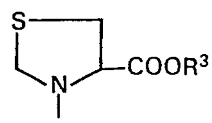

R

Das Verfahren ist dadurch gekennzeichnet, dass man eine Verbindung der Formel II

$$\begin{array}{l} \quad\quad OSO_2CF_3 \\ \quad\ * \ / \\ R(CH_2)_n\text{-}CH \\ \quad\quad \backslash \\ \quad\quad COOR^2 \end{array} \qquad (II)$$

in welcher n, R und $R^2$ wie oben definiert sind, mit einer Verbindung der Formel IV

$$\begin{array}{l} \quad *a \qquad *b \\ R^3OOC\text{-}CH\text{-}N\text{ - }C\text{ -}CH\text{-}NH_2 \\ \quad\ \ | \quad\ | \quad \| \quad | \\ \quad R^4 \ \ R^5 \ \ O \ \ R^1 \end{array} \qquad (IV)$$

in welcher $R^1$, $R^3$, $R^4$ und $R^5$ wie oben definiert sind, oder eine Verbindung der Formel III

$$\begin{array}{l} \quad *a \qquad *b \\ R^3OOC\text{-}CH\text{-}N\text{ - }C\text{ -}CH\text{-}OSO_2CF_3 \\ \quad\ \ | \quad\ | \quad \| \quad | \\ \quad R^4 \ \ R^5 \ \ O \ \ R^1 \end{array} \qquad (III)$$

in welcher $R^1$, $R^3$, $R^4$ und $R^5$ wie oben definiert sind, mit einer Verbindung der Formel V

$$\begin{array}{l} \qquad\quad * \\ R\text{-}(CH_2)_n\text{-}CH\text{-}NH_2 \\ \qquad\quad | \\ \qquad COOR^2 \end{array} \qquad (V)$$

in welcher n, R und $R^2$ wie oben definiert sind, in Gegenwart von mindestens einem Äquivalent einer Base, die nicht mit Verbindungen der Formeln II oder III abreagieren kann, umsetzt, gegebenenfalls Estergruppen hydrolytisch oder hydrogenolytisch abspaltet und gegebenenfalls freie Carboxygruppen in an sich bekannter Weise verestert.

Aus der Literatur (z.B. aus der US-PS 4 350 704 und den EP-A 49 605 und 46 953) sind Verfahren zur Herstellung von Verbindungen der Formel I durch Umsetzung von α-Halogencarbonsäureestern bzw. der entsprechend Tosyloxy- oder Mesyloxy-Verbindungen mit Aminosäureestern oder Dipeptidestern unter nucleophiler Substitution bekannt. Im allgemeinen erfordern diese Umsetzungen eine erhöhte Reaktionstemperatur und die Ausbeuten sind häufig aufgrund der drastischen Reaktionsbedingungen, die Nebenreaktionen begünstigen, gering. Oft ist eine Katalyse mit Silberionen, wie z.B. bei der Umsetzung der α-Halogencarbonsäureester, notwendig, die die Ausbeute verbessert, das Verfahren jedoch wesentlich verteuert. Häufig erhält man bei Einsatz optisch aktiver α-Halogen-, α-Mesyloxy bzw. α-Tosyloxycarbonsäureester racemische Produkte.

In Angew. Chem. 95 (1983) 50 (Effenberger et al.) ist die Herstellung N-substituierter α-Aminosäuren durch Umsetzung von 1 Äquivalent α-Trifluormethansulfonyloxy-carbonsäureester mit 2 Äquivalenten primären Amins in Abwesenheit einer Base beschrieben.

In einem weiteren literaturbekannten Verfahren, das unter anderem Gegenstand der deutschen Patentanmeldung P 3 226 768.1 ist, werden α-Ketoester mit Aminosäureestern und Dipeptidestern zu den entsprechenden Schiff-Basen umgesetzt und diese mit verschiedenen Reduktionsmitteln reduziert. Besonders geeignet ist hier Natriumcyanborhydrid. Bei der Aufarbeitung entsteht hierbei Blausäure, was das Verfahren sehr aufwendig macht. Das beanspruchte Verfahren besitzt die angegebenen Nachteile nicht.

Eine bevorzugte Ausführungsform ist dadurch gekennzeichnet, dass Verbindungen der Formel I hergestellt werden, in der
n 1 oder 2 ist
R Wasserstoff
Alkyl mit 1-8 C-Atomen,

Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, $C_1$-$C_4$)-Acylamino, vorzugsweise ($C_1$-$C_4$)-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben, substituiert sein kann,
mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
$C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen, das wie oben bei R beschrieben substituiert sein kann,
($C_6$-$C_{12}$)-Aryl-($C_1$-$C_4$)-alkyl oder
($C_7$-$C_{13}$)-Aroyl-($C_1$ oder $C_2$)-alkyl,
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann, oder
die Seitenkette einer gegebenenfalls geschützten natürlich vorkommenden α-Aminosäure $R^1$-CH--($NH_2$)-COOH bedeuten,
$R^2$ und $R^3$ gleich oder verschieden sind und Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aryloxy-($C_1$-$C_4$)-alkyl,
($C_6$-$C_{12}$)-Aryloxycarbonyloxy-($C_1$-$C_4$)-alkyl,
Aryl mit 6-12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und
$R^4$ und $R^5$ die oben angegebene Bedeutung haben.

Besonders bevorzugt ist eine Ausführungsform, die dadurch gekennzeichnet ist, dass Verbindungen der Formel I hergestellt werden, in der
n = 1 oder 2 ist,
R ($C_1$ bis $C_6$)-Alkyl, ($C_2$ bis $C_6$)-Alkenyl, ($C_3$ bis $C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$ bis $C_{12}$)-Aryl-($C_1$-$C_4$)-alkoxycarbonylamino-($C_1$-$C_4$)-alkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylendioxy mono-, di- oder trisubstituiert sein kann, oder 3-Indolyl, insbesondere Methyl, Ethyl, Cyclohexyl, tert.-Butoxycarbonylamino-($C_1$-$C_4$)-alkyl, Benzoyloxycarbonylamino-($C_1$-$C_4$)-alkyl oder Phenyl, das durch Phenyl, ($C_1$ bis $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkylamino, Nitro und/oder Methylendioxy mono- oder disubstituiert oder im Falle von Methoxy, trisubstituiert sein kann, bedeutet,
$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$ bis $C_3$)-Alkyl, ($C_2$ oder $C_3$)-Alkenyl, die gegebenenfalls ge-

schützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl, insbesondere aber Wasserstoff, ($C_1$ bis $C_4$)-Alkyl oder Benzyl bedeuten und

$R^4$ und $R^5$ die oben angegebene Bedeutung haben.

Unter Aryl ist hier im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatischen Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b] thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, dass $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Nach dem erfindungsgemässen Verfahren können, je nachdem, welche chiralen Ausgangsverbindungen verwendet wurden, Verbindungen der Formel I erhalten werden, in denen das bei dieser $S_N2$-Reaktion entstandene Chiralitätszentrum in der S- oder R-Konfiguration oder racemisch vorliegt.

Die beim erfindungsgemässen Verfahren stattfindende Reaktion verläuft stereochemisch eindeutig. Diese Tatsache wird auch durch die Untersuchungen des stereochemischen Verlaufs der Reaktion von α-Trifluormethansulfonyloxy-carbonsäureestern mit optisch aktiven Aminen bestätigt [Effenberger et al., Angew. Chem. 95 (1983) 50].

Das nachstehende Schema erläutert den stereochemischen Verlauf der Reaktion beim erfindungsgemässen Verfahren:

Ausgangsverbindungen →

```
        a          b    C
R³OOC-CH-  N  -C-CH-NH-CH-(CH₂)n-R
       |   |   || |      |
       R⁴  R⁵  O R¹      CO₂R²
```

| | | | | |
|---|---|---|---|---|
| (R)-II | + | ($S_a$,$S_b$)-IV | → | ($S_a$,$S_b$,$S_c$)-I |
| (R)-II | + | ($R_a$,$S_b$)-IV | → | ($R_a$,$S_b$,$S_c$)-I |
| (R)-II | + | ($S_a$,$R_b$)-IV | → | ($S_a$,$R_b$,$S_c$)-I |
| (R)-II | + | ($R_a$,$R_b$)-IV | → | ($S_a$,$R_b$,$S_c$)-I |
| (S)-II | + | ($S_a$,$S_b$)-IV | → | ($S_a$,$S_b$,$R_c$)-I |
| (S)-II | + | ($R_a$,$S_b$)-IV | → | ($R_a$,$S_b$,$R_c$)-I |
| (S)-II | + | ($S_a$,$R_b$)-IV | → | ($S_a$,$R_b$,$R_c$)-I |
| (S)-II | + | ($R_a$,$R_b$)-IV | → | ($R_a$,$R_b$,$S_c$)-I |
| ($S_a$,$S_b$)-III | + | (R)-V | → | ($S_a$,$R_b$,$R_c$)-I |
| ($R_a$,$R_b$)-III | + | (R)-V | → | ($R_a$,$R_b$,$R_c$)-I |
| ($S_a$,$R_b$)-III | + | (R)-V | → | ($S_a$,$S_b$,$R_c$)-I |
| ($R_a$,$R_b$)-III | + | (R)-V | → | ($R_a$,$S_b$,$R_c$)-I |
| ($S_a$,$S_b$)-III | + | (S)-V | → | ($S_a$,$R_b$,$S_c$)-I |
| ($R_a$,$S_b$)-III | + | (S)-V | → | ($R_a$,$R_b$,$S_c$)-I |
| ($S_a$,$R_b$)-III | + | (S)-V | → | ($S_a$,$S_b$,$S_c$)-I |
| ($R_a$,$R_b$)-III | + | (R)-V | → | ($R_a$,$S_b$,$S_c$)-I |

Besonders vorteilhaft können die folgenden Verbindungen nach dem erfindungsgemässen Verfahren erhalten werden.

N-(1-S-Carbethoxy-3-phenylpropyl)-S-alanyl-S-prolin-benzylester

N-(1-R-Carbethoxy-3-phenyl-propyl)-S-alanyl-S-prolin-benzylester

N-(1-R,S-Carbethoxy-3-phenyl-propyl)-S-alanyl-S-prolin-benzylester

N-(1-S-Carbethoxy-3-cyclohexyl-propyl)-S-alanyl-S-prolin-benzylester

N-(1-R-Carbethoxy-3-cyclohexyl-propyl)-S-alanyl-S-prolin-benzylester

N-(1-R,S-Carbethoxy-3-cyclohexyl-propyl)-S-alanyl-S-prolin-benzylester

N-(1-S-Carbethoxy-3-phenyl-propyl)-$N_ε$-benzyloxycarbonyl-S-lysyl-S-prolin-tert. butylester

N-(1-S-Carbethoxy-3-phenyl-propyl)-S-tyrosyl-S-prolin benzylester

N-(1-S-Carbethoxy-3-phenyl-propyl)-O-methyl-S-tyrosyl-S-prolin-benzylester

N-(1-S-Carbethoxy-3-phenyl-propyl)-O-ethyl-S-tyrosyl-S-prolin-benzylester

N-(1-S-Carbethoxy-3-phenyl-propyl)-S-alanyl-S-pipecolsäure-benzylester

N-(1-S-Carbethoxy-3-cyclohexyl-propyl)-S-alanyl-S-pipecolsäurebenzylester

N-(1-S-Carbethoxy-3-phenyl-propyl)-S-alanyl-S-1,2,3,4-tetrahydroisochinolin-3 carbonsäure-tert. butylester

N-(1-S-Carbethoxy-3-cyclohexyl-propyl)-S-alanyl-S-1,2,3,4-tetrahydroisochinolin-3-carbonsäure-tert. butylester

N-(1-S-Carbethoxy-3-phenyl-propyl)-$N_ε$-benzylcarbonyl-S-lysyl-S-1,2,3,4-tetrahydroisochinolin-3-carbonsäure-tert. butylester

N-(1-S-Carbethoxy-3-phenyl-propyl)-O-ethyl-S-tyrosyl-S-1,2,3,4-tetrahydroisochinolin-3-carbonsäure-tert. butylester

N-(1-S-Carbethoxy-3-phenyl-propyl)-S- alanyl-3S-decahydroisochinolin-3-carbonsäure-benzylester

N-(1-S-Carbethoxy-3-phenyl-propyl)-S-alanyl-(2S,3aS,7aS)-octahydroindol-2-carbonsäure-benzylester

N-(1-S-Carbethoxy-3-cyclohexyl-propyl)-S-alanyl-(2S,3aS,7aS)-octahydroindol-2-carbonsäure-benzylester

N-(1-S-Carbethoxy-3-phenyl-propyl)-$N_ε$-benzyloxycarbonyl-S-lysyl-(2S,3aS,7aS)-octahydroindol-2--carbonsäure-tert. butylester

N-(1-S-Carbethoxy-3- cyclohexyl-propyl)-$N_\varepsilon$-benzyloxycarbonyl-S-lysyl-(2S,3aS,7aS)-octahydroindol-2-carbonsäure-tert. butylester

N-(1-S-Carbethoxy-3-phenyl-propyl)-O-methyl-S--tyrosyl-(2S,3aS,7aS)-octahydroindol-2-carbonsäure-benzylester

N-(1-S-Carbethoxy-3-phenyl-propyl)-O-ethyl-S--tyrosyl-(2S,3aS,7aS)-octahydroindol-2-carbonsäure-benzylester

N-(1-S-Carbethoxy-4,4-dimethylphenyl)-S-alanyl-(2S,3aS,7aS)-octahydroindol-2-carbonsäure-benzylester

N-[1-S-Carbethoxy-3-(4-fluorphenyl)-propyl]-S-alanyl-(2S,3aS,7aS)-octahydroindol-2-carbonsäure-benzylester

N-[1-S-Carbethoxy-3-(4-methoxyphenyl)-propyl]-S-alanyl-(2S,3aS,7aS)-octahydroindol-2-carbonsäure-benzylester

N-[1-S-Carbethoxy-3-(3,4-dimethoxyphenyl)-propyl]-S-alanyl-(2S,3aS,7aS)-octahydroindol-2-carbonsäure-benzylester

N-(1-S-Carbethoxy-3-cyclopentylpropyl)-S-alanyl-(2S,3aS,7aS)-octahydroindol-2-carbonsäure benzylester

N-(1-S-Carbethoxy-3-phenyl-propyl)-S-alanyl-(2S, 3aR,7aS)-octahydroindol-2-carbonsäure-benzylester

N-(1-S-Carbethoxy-3-cyclohexyl-propyl)-S-alanyl-(2S,3aR,7aS)-octahydroindol-2-carbonsäure benzylester

N-(1-S-Carbethoxy-3-phenyl-propyl)-$N_\varepsilon$-benzyloxycarbonyl-S-lysyl(2S,3aR,7aS)-octahydroindol-2--carbonsäure tert. butylester

N-(1-S-Carbethoxy-3-cyclohexyl-propyl)-$N_\varepsilon$-benzyloxycarbonyl-S-lysyl-(2S,3aR;7aS)-octahydroindol-2-carbonsäure-benzylester

N-(1-S-Carbethoxy-3-phenyl-propyl)-O-ethyl-S--tyrosyl-(2S,3aR,7aS)-octahydroindol-2-carbonsäure-benzylester

N-(1-S-Carbethoxy-3-phenyl-propyl)-S- alanyl-(2S, 3aR,7aR)-octahydroindol-2-carbonsäure-benzylester

N-(1-S-Carbethoxy-3-phenyl-propyl)-$N_\varepsilon$-benzyloxycarbonyl-S-lysyl-(2S,3aR,7aR)-octahydroindol-2--carbonsäure-tert. butylester

N-(1-S-Carbethoxy-3-cyclohexyl-propyl)-S-alanyl-(2S,3aR,7aR)-octahydroindol-2-carbonsäure-benzylester

N-(1-S-Carbethoxy-3-cyclohexyl-propyl)-O-ethyl--S-tyrosyl-(2S,3aR,7aR)-octahydroindol-2-carbonsäure-benzylester

N-(1-S-Carbethoxy-3-phenyl-propyl)-S-alanyl-(2S, 3aS,7aR)-octahydroindol-2-carbonsäure-benzylester

N-(1-S-Carbethoxy-3-phenyl-propyl)-S-alanyl-(2S, 3aS,6aS)-octahydrocyclopenta[b]pyrrol-2-carbonsäure-benzylester

N-(1-S-Carbethoxy-3-cyclohexyl-propyl)-S-alanyl-(2S,3aS,6aS)-octahydrocyclopenta[b]pyrrol-2-carbonsäure-benzylester

N-(1-S-Carbethoxy-3-phenyl-propyl)-$N_\varepsilon$-benzyloxycarbonyl-S-lysyl-(2S,3aS,6aS)-octahydrocyclopenta[b]pyrrol-2-carbonsäure-tert. butylester

N-(1-S-Carbethoxy-3-cyclohexyl-propyl)-$N_\varepsilon$-benzyloxycarbonyl-S-lysyl-(2S,3aS,6aS)-octahydrocyclopenta[b]pyrrol-2-carbonsäure-tert. butylester

N-(1-S-Carbethoxy-3-phenyl-propyl)-O-methyl-S--tyrosyl-(2S,3aS,6aS)-octahydrocyclopenta[b]-pyrrol-2-carbonsäure-benzylester

N-(1-S-Carbethoxy-3-phenyl-propyl)-O-ethyl-S--tyrosyl-(2S,3aS,6aS)-octahydrocyclopenta[b]-pyrrol-2-carbonsäure-benzylester

N-(1-S-Carbethoxy-3-[4-fluor-phenyl]-propyl)-S--alanyl-(2S,3aS,6aS)-octahydrocyclopenta[b]pyrrol-2-carbonsäure-benzylester

N-(1-S-Carbethoxy-3-[4-methoxyphenyl]-propyl)-S--alanyl-(2S,3aS,6aS)-octahydrocyclopenta[b]pyrrol-2-carbonsäure-benzylester

N-(1-S-Carbethoxy-3-cyclopentylpropyl)-S-alanyl-(2S,3aS,6aS)-octahydrocyclopenta[b]pyrrol-2-carbonsäure-benzylester

N-(1-S-Carbethoxy-3-phenyl-propyl)-S-alanyl-(2S, 3aR,6aR)-octahydrocyclopenta[b]pyrrol-2-carbonsäure-benzylester

N-(1-S-Carbethoxy-3-cyclohexylpropyl)-S- alanyl-(2S,3aR,6aR)-octahydrocyclopenta[b]pyrrol-2-carbonsäure-benzylester

N-(1-S-Carbethoxy-3-phenyl-propyl)-$N_\varepsilon$-benzyloxycarbonyl-S-lysyl-(2S,3aR,6aR)-octahydrocyclopenta[b]pyrrol-2-carbonsäure-tert. benzylester

N-(1-S-Carbethoxy-3-phenyl-propyl)-S-alanyl-(2S, 3aR,6aS)-octahydrocyclopenta[b]pyrrol-2-carbonsäure-benzylester

N-(1-S-Carbethoxy-3-cyclohexylpropyl)-S-alanyl-(2S,3aR,6aS)-octahydrocyclopenta[b]pyrrol-2-carbonsäure-benzylester

N-(1-S-Carbethoxy-3-phenyl-propyl)-$N_\varepsilon$-benzyloxycarbonyl-S-lysyl-(2S,3aR,6aS)-octahydrocyclopenta[b]pyrrol-2-carbonsäure-tert. butylester

N-(1-S-Carbethoxy-3-cyclohexylpropyl)-$N_\varepsilon$-benzyloxycarbonyl-S-lysyl-(2S,3aR,6aS)-octahydrocyclopenta[b]pyrrol-2-carbonsäure-tert. butylester

N-(1-S-Carbethoxy-3-phenyl-propyl)-O-ethyl-S--tyrosyl-(2S,3aR,6aS)-octahydrocyclopenta[b]pyrrol-2-carbonsäure-benzylester

N-(1-S-Carbethoxy-3-cyclopentyl-propyl)-S-alanyl-2-(2S,3aR,6aS)-octahydrocyclopenta[b]pyrrol-2--carbonsäure-benzylester

N-(1-S-Carbethoxy-3-phenyl-propyl)-S-alanyl-S-2--azabicyclo[2.2.2]octan-3-carbonsäure-benzylester

N-(1-S-Carbethoxy-3-phenyl-propyl)-O-ethyl-S--tyrosyl-S-2-azabicyclo[2.2.2]-octan-3-carbonsäure-benzylester

N-(1-S-Carbethoxy-3-cyclohexyl-propyl)-S-alanyl--S-2-azabicyclo[2.2.2]octan-3-carbonsäure-benzylester

N-(1-S-Carbethoxy-3-cyclohexyl-propyl)-$N_\varepsilon$-benzyloxycarbonyl-S-2-azabicyclo[2.2.2]-octan-3-carbonsäure-tert. butylester

N-(1-S-Carbethoxy-3-phenyl-propyl)-$N_\varepsilon$-benzyloxycarbonyl-S-lysyl-S-2-azabicyclo[2.2.2]-octan-3--carbonsäure-tert. butylester

N-(1-S-Carbethoxy-3-cyclopentylpropyl)-S-alanyl--S-2-azabicyclo[2.2.2]-octan-3-carbonsäure-benzylester

N-(1-S-Carbethoxy-3-phenyl-propyl)-S-alanyl-3S--exo-2-azabicyclo[2.2.1]heptan-3-carbonsäure--benzylester

N-(1-S-Carbethoxy-3-cyclohexylpropyl)-S-alanyl-3S-exo-2-azabicyclo[2.2.1]heptan-3-carbonsäure-benzylester
N-(1-Carbethoxy-3-phenyl-propyl)-$N_\varepsilon$-benzyloxy-carbonyl-S-lysyl-3S-exo-2-azabicyclo[2.2.1]heptan-3-carbonsäure-tert. butylester
N-(1-S-Carbethoxy-3-phenyl-propyl)-S-alanyl-3S-endo-2-azabicyclo[2.2.1]heptan-3-carbonsäure-benzylester
N-(1-S-Carbethoxy-3-cyclohexylpropyl)-S-alanyl-3S-endo-2-azabicyclo[2.2.1]heptan-3-carbonsäure-benzylester
N-(1-S-Carbethoxy-3-phenyl-propyl)-$N_\varepsilon$-benzyloxycarbonyl-S-lysyl-3S-endo-2-azabicyclo[2.2.1]-heptan-3-carbonsäure-tert. butylester
N-(1-S-Carbethoxy-3-phenyl-propyl)-O-ethyl-S-tyrosyl-3S-endo-2-azabicyclo[2.2.1]heptan-3-carbonsäure-benzylester
N-(1-S-Carbethoxy-3-phenyl-propyl)-S-alanyl-2-azaspiro[4,5]decan-3S-carbonsäure-benzylester
N-(1-S-Carbethoxy-3-phenyl-propyl)-O-ethyl-S-tyrosyl-2-azaspiro[4,5]decan-3-S-carbonsäure-benzylester
N-(1-S-Carbethoxy-3-phenyl-propyl)-$N_\varepsilon$-benzyloxycarbonyl-S-lysyl-2-azaspiro[4,5]decan-3-S-carbonsäure-tert.-butylester
N-(1-S-Carbethoxy-3-cyclohexylpropyl)-S-alanyl-2-azaspiro[4,5]decan-3-S-carbonsäure-benzylester
N-(1-S-Carbethoxy-3-ccyclohexylpropyl)-$N_\varepsilon$-benzyloxycarbonyl-S-lysyl-2-azaspiro[4,5]decan-3 S-carbonsäure-tert.-butylester
N-(1-S-Carbethoxy-3-phenyl-propyl)-S-alanyl-2-azaspiro[4,4]nonan-3-S-carbonsäure-benzylester
N-(1-S-Carbethoxy-3-phenyl-propyl)-O-ethyl-S-tyrosyl-2-azaspiro[4,4]nonan-3-S-carbonsäure benzylester
N-(1-S-Carbethoxy-3-phenyl-propyl)-$N_\varepsilon$-tert.butoxycarbonyl-S-lysyl-2-azaspiro[4,4]nonan-3-S-carbonsäure-benzylester
N-(1-S-Carbethoxy-3-cyclohexyl-propyl)-S-alanyl-2-azaspiro[4,4]nonan-3-S-carbonsäure-benzylester
N-(1-S-Carbethoxy-3-cyclopentyl-propyl)-S-alanyl-2-azaspiro[4,4]nonan-3S-carbonsäure-benzylester
N-(1-S-Carbethoxy-3-cyclopentyl-propyl)-$N_\varepsilon$-tert.-butoxycarbonyl-S-lysyl-2-azaspiro[4,4]nonan-3-S-carbonsäure-benzylester
N-(1-S-Carbethoxy-3-phenyl-propyl)-S-alanyl-spiro-[bicyclo[2.2.1]heptan-2,3'-pyrrolidin]-5'-S-carbonsäure-benzylester
N-(1-S-Carbethoxy-3-phenyl-propyl)-O-ethyl-S-tyrosyl-spiro[bicyclo[2.2.1]heptan-2,3'-pyrrolidin]-5'-S-carbonsäure-benzylester
N-(1-S-Carbethoxy-3-phenyl-propyl)-$N_\varepsilon$-benzyloxycarbonyl-S-lysyl-spiro[bicyclo[2.2.1]heptan-2,3'-pyrrolidin]-5'-S-carbonsäure-tert. butylester
N-(1-S-Carbethoxy-3-cyclohexyl-propyl)-S-alanyl-spiro[bicyclo]2.2.1]heptan-2,3'-pyrrolidin]-5'-S-carbonsäure-benzylester
N-(1-S-Carbethoxy-3-cyclohexyl-propyl)-$N_\varepsilon$-tert.-butoxycarbonyl-S-lysyl-spiro[bicyclo[2.2.1]heptan-2,3'-pyrrolidin]-5'-S-carbonsäure-benzylester
N-(1-S-Carbethoxy-3-phenyl-propyl)-S-alanyl-spiro-[bicyclo[2.2.2]octan-2,3'-pyrrolidin]-5'-S-carbonsäure-benzylester
N-(1-S-Carbethoxy-3-phenyl-propyl)-O-ethyl-S-tyrosyl-spiro[bicyclo[2.2.2]octan-2,3'-pyrrolidin]-5'-S-carbonsäure-benzylester
N-(1-S-Carbethoxy-3-phenyl-propyl)-$N_\varepsilon$-tert.-butoxycarbonyl-S-lysyl-spiro[bicyclo[2.2.2]octan-2,3'-pyrrolidin]-5'-S-carbonsäure-benzylester
N-(1-S-Carbethoxy-3-cyclohexyl-propyl)-S-alanyl-spiro[bicyclo[2.2.2]octan-2,3'-pyrrolidin]-5'-S-carbonsäure-benzylester
N-(1-S-Carbethoxy-3-phenyl-propyl)-S-alanyl-azatricyclo[4,3,0,$1^{6,9}$]decan-3-S-carbonsäure-benzylester
N-(1-S-Carbethoxy-3-phenyl-propyl)-O-ethyl-S-tyrosyl-2-azatricyclo[4.3.0.$1^{6,9}$]decan-3-S-carbonsäure-benzylester
N-(1-S-Carbethoxy-3-phenyl-propyl)-$N_\varepsilon$-benzyloxycarbonyl-S-lysyl-2-azatricyclo[4.3.0.$1^{6,9}$]decan-3-S-carbonsäure-tert.-butylester
N-(1-S-Carbethoxy-3-cyclohexyl-propyl)-S-alanyl-2-azatricyclo[4,3,0,$1^{6,9}$]decan-3-S-carbonsäure-benzylester
N-(1-S-Carbethoxy-3-phenyl-propyl)-$N_\varepsilon$-benzyloxycarbonyl-S-lysyl-2-azatricyclo[4.3.0.$1^{6,9}$]decan-3-S-carbonsäure-tert. butylester
N-(1-S-Carbethoxy-3-phenyl-propyl)-S-alanyl-decahydro-cyclohepta[b]pyrrol-2-S-carbonsäure-benzylester
N-(1-S-Carbethoxy-3-phenyl-propyl)-O-ethyl-S-tyrosyl-decahydrocyclohepta[b]pyrrol-2-S-carbonsäure-benzylester
N-(1-S-Carbethoxy-3-phenyl-propyl)-$N_\varepsilon$-benzyloxycarbonyl-S-lysyl-decahydrocyclohepta[b]pyrrol-2-S-carbonsäure-tert. butylester
N-(1-S-Carbethoxy-3-cyclohexyl-propyl)-S-alanyl-decahydrocyclohepta[b]pyrrol-2-S-carbonsäure-benzylester
N-(1-S-Carbethoxy-3-cyclohexyl-propyl)-$N_\varepsilon$-tert.-butoxycarbonyl-S-lysyl-decahydrocyclohepta[b]pyrrol-2-S-carbonsäure-benzylester
N-(1-S-Carbethoxy-3-phenyl-propyl)-S-alanyl-trans-octahydroisoindol-1-S-carbonsäure-benzylester
N-(1-S-Carbethoxy-3-phenyl-propyl)-S-alanyl-cis-octahydroisoindol-1-S-carbonsäure-benzylester
N-(1-S-Carbethoxy-3-cyclohexyl-propyl)-S-alanyl-trans-octahydroisoindol-1-S-carbonsäure-benzylester
N-(1-S-Carbethoxy-3-cyclohexyl-propyl)-S-alanyl-cis-octahydroisoindol-1-S-carbonsäure-benzylester
N-(1-S-Carbethoxy-3-phenyl-propyl)-S-alanyl-cis-octahydrocyclopenta[c]pyrrol-1-S-carbonsäure-benzylester
N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-cyclohexyl-propyl)-$N_\varepsilon$-tert.-butoxycarbonyl-S-lysyl-cis-octahydrocyclopenta-[c]pyrrol-1-S-carbonsäure-benzylester
N-(1-S-Carbethoxy-3-phenyl-propyl)-S-alanyl-2,3,3a,4,5,7a-hexahydroindol-2-S-carbonsäure-tert.-butylester
N-(1-S-Carbethoxy-3-cyclohexyl-propyl)-O-ethyl-S-tyrosyl-2,3,3a,4,5,7a-hexahydroindol-2-S-carbonsäure-tert.-butylester

N-(1-S-Carbethoxy-3-cyclohexyl-propyl)-S-alanyl-2,3,3a,4,5,7a-hexahydroindol-2-S-carbonsäure-tert.-butylester
N-(1-S-Carbethoxy-3-phenyl-propyl)-S-alanyl-thiazolidin-5-S-carbonsäure-tert.-butylester
N-(1-S-Carbethoxy-3-cyclohexyl-propyl)-S-alanyl-thiazolidin-5-S-carbonsäure-tert.-butylester
N-(1-S-Carbethoxy-3-phenyl-propyl)-$N_\varepsilon$-benzyloxycarbonyl-lysyl-thiazolidin-5-S-carbonsäure-tert.-butylester.

Die Erfindung betrifft auch Verbindungen der Formel III, in welcher

$R^1$ Wasserstoff oder ($C_1$ bis $C_6$)-Alkyl, das gegebenenfalls durch Amino, ($C_1$ bis $C_6$)-Acylamino, vorzugsweise Boc- oder ($C_1$-$C_6$)-Alkanoylamino, oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl- oder teilhydriertes Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$-$C_2$)alkyl,die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure,

$R_3$ Wasserstoff, ($C_1$ bis $C_6$)-Alkyl, ($C_2$ bis $C_6$)-Alkenyl oder ($C_6$-$C_{12}$)-Aryl-($C_1$ bis $C_4$)-alkyl bedeuten,

$R^4$ und $R^5$ die bei Verbindungen der Formel I angegebene Bedeutung haben.

Bevorzugt sind solche Verbindungen der Formel III, in welcher

$R^1$ Wasserstoff, ($C_1$ bis $C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl

$R^3$ Wasserstoff, ($C_1$ bis $C_4$)-Alkyl oder Benzyl bedeuten,

und

$R^4$ und $R^5$ die bei Verbindungen der Formel I angegebene Bedeutung haben,

insbesondere aber Verbindungen der Formeln III, in welcher

$R^1$ Methyl, Ethyl, Phenyl, die gegebenenfalls acylierte Seitenkette von Lysin, Benzyl oder die ($C_1$-$C_6$)-O-alkylierte Seitenkette von Tyrosin,

$R^3$ Wasserstoff, Methyl, Ethyl, tert.-Butyl oder Benzyl bedeuten und

$R^4$ und $R^5$ die bei Verbindungen der Formel I angegebene Bedeutung haben.

Die Trifluormethansulfonate der Formeln II und III erhält man, indem man α-Hydroxycarbonsäurederivate der Formeln VI bzw. VII

```
             * OH
R-[CH2]n-CH<                    (VI)
               COR2
               ||
               O
```

```
         *a       *b
R3OOC-CH- N -C - CH-OH          (VII)
       |   |  ||   |
       R4  R5 O    R1
```

in welchen n, R, $R^1$, $R^2$, $R^3$, $R^4$ und $R^5$ die oben genannten Bedeutungen haben, mit einem Trifluormethansulfonierungsmittel, wie z.B. Trifluormethansulfonsäureanhydrid oder Trifluormethansulfonylchlorid in einem inerten Lösungsmittel umsetzt.

Die Dipeptidderivate der Formel IV und die Hydroxycarbonsäureamide der Formel VII erhält man nach an sich bekannten Methoden der Peptidchemie unter Verwendung geeigneter Schutzgruppentechnik aus den jeweiligen Aminosäuren bzw. Hydroxysäuren.

Um die bei der Reaktion entstehende Säure abzufangen, ist es vorteilhaft, die Reaktion in Gegenwart einer Base durchzuführen. Dafür eignen sich anorganische Salze wie Carbonate (z.B. $K_2CO_3$, $Na_2CO_3$, $NaHCO_3$), $Na_2SO_4$ oder organische Basen wie z.B. Triethylamin, Pyridin. Man kann die Base in stöchiometrischer Menge oder auch im Überschuss verwenden.

Als Lösungsmittel sind diejenigen geeignet, die nicht mit dem Trifluormethansulfonierungsmittel und den Trifluormethansulfonsäurederivaten reagieren können. Dies sind z.B. Lösungsmittel wie Methylenchlorid, Chloroform, Teatrachlorkohlenstoff, oder andere halogenierte Kohlenwasserstoffe oder auch Kohlenwasserstoffe wie z.B. Hexan. Die Reaktion kann in einem Temperaturbereich zwischen -80°C und +80°C ausgeführt werden. Besonders vorteilhaft ist die Reaktion in Methylenchlorid, Chloroform oder Tetrachlorkohlenstoff, wobei Trifluormethansulfonsäureanhydrid in Gegenwart von Pyridin mit dem α-Hydroxycarbonsäurederivat bei Temperaturen zwischen −80°C und Raumtemperatur umgesetzt wird. Trifluormethansulfonsäureanhydrid kann auch im Überschuss verwendet werden.

Werden optisch aktive Verbindungen der Formel VI oder VII eingesetzt, so bleibt bei der Überführung in die Verbindungen der Formeln II oder III die Konfiguration am chiralen Kohlenstoffatom erhalten.

Die Trifluormethansulfonsäurederivate der Formeln II bzw. III reagieren mit Aminosäureestern der Formel IV bzw. V glatt zu Verbindungen der Formel I. Um die entstehende Trifluormethansulfonsäure abzufangen, wird die Reaktion zweckmässig in Gegenwart eines Äquivalents einer Base durchgeführt, die nicht mit Verbindungen der Formeln II oder III abreagieren kann. Als vorteilhaft haben sich tert. Amine wie Triethylamin oder Pyridin erwiesen. Auch die Aminosäurederivate selbst können als Säurefänger dienen. Geeignet sind auch anorganische Salze wie z.B. $Na_2CO_3$, $K_2CO_3$, $NaHCO_3$, $Na_2SO_4$.

Die Reaktion wird in einem aprotischen polaren Lösungsmittel oder unpolaren Lösungsmittel durchgeführt. Geeignete Lösungsmittel sind z.B. Methylenchlorid, Chloroform, Tetrachlorkohlenstoff, Dimethylformamid, Essigsäureethylester, Dimethoxyethan, Hexan, Ether, Tetrahydrofuran.

Die Reaktionstemperatur liegt im Bereich zwischen −80 und +150°C. Besonders vorteilhaft hat sich −20 bis +80°C erwiesen.

Die Aufarbeitung gestaltet sich sehr einfach. Das Lösungsmittel wird mit Wasser gewaschen, um die gebildeten Salze zu entfernen. Die organische Lösung wird nach dem Trocknen eingeengt, wobei die Verbindungen der Formel I rein anfallen, die erforderlichenfalls nach den allgemeinen Reinigungsmethoden, wie z.B. u.a. Filtration oder Chromatographie über Kieselgel, hoch gereinigt werden können.

Werden optisch reine Verbindungen der Formeln II oder III in die Reaktion eingesetzt, so erfolgt die Substitution des Trifluormethansulfonsäureesters durch die Aminosäurederivate der Formel IV oder V unter Konfigurationsumkehr. Man erhält ohne Racemisierung aus optisch reinen Ausgangsprodukten optisch reine Endprodukte. Ein Diastereomerengemisch erhält man, wenn z.B. racemische Verbindungen der Formel II oder III mit optisch reinen Aminosäurederivaten oder umgekehrt, oder racemische Verbindungen der Formel II oder III mit racemischen Aminosäurederivaten umgesetzt werden. Die entstandenen Diastereomeren können nach den allgemein üblichen Trennmethoden, wie z.B. fraktionierte Kristallisation der Salze oder Säulenchromatographie über Kieselgel getrennt werden. Auch wenn eine der Ausgangskomponenten racemisch ist, bietet das erfindungsgemässe Verfahren aufgrund der hohen Ausbeuten und Reinheit grosse Vorteile gegenüber bekannten Verfahren.

Die Verbindungen der Formel I sind Hemmstoffe des Angiotensin Converting Enzymes (ACE) beziehungsweise Zwischenprodukte bei der Herstellung von solchen Hemmstoffen und können zur Bekämpfung des Bluthochdrucks verschiedener Genese eingesetzt werden. Solche Verbindungen sind beispielsweise bekannt aus US-PS 4 350 633, US-PS 4 344 949, US-PS 4 294 832, US-PS 4 350 704, EP-A 50 800, EP-A 31 741, EP-A 51 020, EP-A 49 658, EP-A 49 605, EP-A 29 488, EP-A 49 953 und EP-A 52 870. Sie sind weiterhin Gegenstand der deutschen Patentanmeldungen P 3 226 768.1, P 3 151 690.4, P 3 210 496.0, P 3 211 397.8, P 3 211 676.4, P 3 227 055.0, P 3 242 151.6, P 3 246 503.3 und P 3 246 757.5.

Die nachfolgenden Beispiele sollen das erfindungsgemässe Verfahren erläutern, ohne die Erfindung auf die hier stellvertretend genannten Substanzen zu beschränken.

*Beispiel 1*

*N-(1-Carbethoxy-3-phenyl-propyl)-S-alanyl-1,2,3,4-tetrahydroisochinolin-3-S-carbonsäure-tert. butylester*

*(Diastereomere A 1 und B 1)*

1,62 g (3,4 mmol) S-Alanyl-1,2,3,4-tetrahydroisochinolin-3-S-carbonsäure-tert. butylester p-Toluolsulfonat werden in 50 ml 10%iger Natriumcarbonatlösung gelöst und dreimal mit Dichlormethan extrahiert; der Extrakt wird mit Natriumsulfat getrocknet und eingeengt. Der Rückstand wird zusammen mit 3,4 mmol Triethylamin in 10 mol trockenem Dichlormethan gelöst; bei 20°C gibt man 1,16 g (3,4 mmol) 4-Phenyl-2-R, S-trifluormethansulfonyloxybuttersäureethylester gelöst in 5 ml trockenem Dichlormethan tropfenweise zu. Nach 1 Stunde bei 20°C wird mit Wasser gewaschen, über Natriumsulfat getrocknet und eingeengt. Die Diastereomeren A 1 und B 1 trennt man durch Chromatographie an Kieselgel mit Essigester/Cyclohexan (1:2) als Laufmittel.

Diastereomer A 1: 0,57 g (35% Ausbeute)

| | | |
|---|---|---|
| $^1$H-NMR-Daten ($CDCl_3$): | 7,15 | (s, 9H) |
| | 5,5-5,2 | (m, 1H) |
| | 4,6 | (s, 2H) |
| | 4,4-3,5 | (m, 4H) |
| | 3,4-1,6 | (m, 6H) |
| | 1,4-1,05 | (d+t, 6H) |
| | 1,25 | (s, 9H) ppm |

Diastereomer B 1: 0,51 g (31% Ausbeute)

| | | |
|---|---|---|
| $^1$H-NMR-Daten ($CDCl_3$): | 7,2 | (s, 9H) |
| | 5,45-5,0 | (2dd, 1H) |
| | 4,71+4,65 | (2s, 2H) |
| | 4,4-3,5 | (m, 4H) |
| | 3,3-1,6 | (m, 6H) |
| | 1,5-1,1 | (d+t, 6H) |
| | 1,27 | (s, 9H) ppm. |

Der benötigte 2-R,S-Trifluormethansulfonyloxy-4-phenylbuttersäureethylester wird erhalten, indem man zu einer Lösung von 6,24 g (30 mmol) 2-R,S-Hydroxy-4-phenylbuttersäureethylester in 30 ml trockenem Dichlormethan unter Rühren bei 0°C innerhalb einer Stunde eine Lösung von 2,37 g Pyridin und 9,73 g Trifluormethansulfonsäureanhydrid in 8 ml Dichlormethan tropft, 15 Minuten bei 0°C nachrührt, den Niederschlag absaugt, das Filtrat einengt und über Kieselgel filtriert. Man erhält 8,6 g des Esters.

Rf-Wert: 0,37 ($SiO_2$, Cyclohexan/Essigester 4:1).

*Beispiel 2*

*N-(1-S-Carbethoxy-3-phenyl-propyl)-S-alanyl-1,2,3,4-tetrahydroisochinolin-3-S-carbonsäure-tert. butylester*

a) 2-R-Trifluormethansulfonyloxy-4-phenylbuttersäureethylester

Die Verbindung wird analog der Herstellungsvorschrift in Beispiel 1 aus 2-R-Hydroxy-4-phenylbuttersäureethylester und Trifluormethansulfonsäureanhydrid erhalten. Der Ethylester wird aus 2-R-Hydroxy-4-phenylbuttersäure [Biquard, Annales de Chimie 20, S. 145 (1933)] und absolutem Ethanol durch Einleiten von trockenem Chlorwasserstoffgas in die auf dem Wasserbad erwärmte Lösung, analog der Vorschrift von Biquard, Annales de Chimie 20, 147 (1933), hergestellt.

Rf: 0,11 ($SiO_2$, Cyclohexan/Essigester 9:1)
Ausbeute: 90%

b) N-(1-S-Carbethoxy-3-phenyl-propyl)-S-alanyl-1,2,3,4-tetrahydroisochinolin-3-S-carbonsäure-tert. butylester

Analog Beispiel 1 wird 2-R-Trifluormethansulfonyloxy-4-phenylbuttersäureethylester mit S-Alanyl-1,2,3,4-tetrahydroisochinolin-3-S-carbonsäure-tert. butylester umgesetzt. Unter Konfigurationsumkehr im Buttersäureteil wird die gewünschte S,S,S-Verbindung in 84% Ausbeute erhalten. Die physikalischen Daten stimmen mit denen von Diastereomer B 1, Beispiel 1 überein.

*Beispiel 3*

*N-(1-S-Carbethoxy-3-phenyl-propyl)-O-methyl-S--tyrosyl-S-prolin-benzylester*

Hergestellt aus 2-R-Trifluormethansulfonyloxy-4--phenylbuttersäureethylester und O-Methyl-S-tyrosyl-S-prolinbenzylester nach dem in Beispiel 2 b beschriebenen Verfahren.
m/e: 572.

*Beispiel 4*

*N-(1-S-Carbethoxy-3-phenyl-propyl)-O-ethyl-S--tyrosyl-(2S,3aS,7aS)-octahydroindol-2-carbonsäure-benzylester*

Hergestellt aus 2-R-Trifluormethansulfonyloxy-4--phenylbuttersäureethylester und O-Ethyl-S-tyrosyl-(2S,3aS,7aS)-octahydroindol-2-carbonsäurebenzylester nach dem in Beispiel 2 b beschriebenen Verfahren.
m/e: 640.

*Beispiel 5*

*N-(1-S-carbethoxy-3-phenyl-propyl)-$N_\varepsilon$-benzyloxycarbonyl-S-lysyl-(2S,3aS,6aS)-Octahydrocyclopenta[b]pyrrol-2S-carbonsäure-tert. butylester*

Hergestellt aus 2-R-Trifluormethansulfonyloxy-4--phenylbuttersäureethylester und $N_\varepsilon$-Benzyloxycarbonyl-S-lysyl-(2S,3aS,6aS)-octahydrocyclopenta[b]pyrrol-2-carbonsäure-tert. butylester analog dem in Beispiel 2 b beschriebenen Verfahren.
m/e: 663.

*Beispiel 6*

*N-(1-S-Carbethoxy-3-cyclohexylpropyl)-S-alanyl--(2S,3aS,6aS)-octahydrocyclopenta[b]pyrrol-2-carbonsäure-benzylester*

a) 2-R-Trifluormethansulfonyloxy-4-cyclohexyl--buttersäure-ethylester

Hergestellt aus 2-R-Hydroxy-4-cyclohexyl-buttersäureethylester nach der in Beispiel 2 a beschriebenen Verfahrensweise.

| | | |
|---|---|---|
| $^1$H-NMR-Daten ($CDCl_3$) δ: | 5,05 | (t, 1H) |
| | 4,25 | (q, 2H) |
| | 2,3-0,9 | (m, 15H) |
| | 1,3 | (t, 3H) ppm. |

b) N-(1-S-Carbethoxy-3-cyclohexylpropyl)-S-alanyl-(2S,3aS,6aS)-octahydrocyclopenta[b]pyrrol-2-carbonsäure-benzylester

Hergestellt aus 2-R-Trifluormethansulfonyloxy-4--cyclohexylbuttersäureethylester und S-Alanyl-(2S, 3aS,6aS)-octahydrocyclopenta[b]pyrrol-2-carbonsäurebenzylester nach dem in Beispiel 2 b beschriebenen Verfahren.

| | | |
|---|---|---|
| $^1$H-NMR-Daten ($CDCl_3$) δ: | 7,33 | (s, 5H) |
| | 5,15 | (s, 2H) |
| | 4,65 | (dd, 1H) |
| | 4,2 | (q, 2H) |
| | 3,8-1,0 | (m, 27H) |
| | 1,3 | (t, 3H) ppm. |

*Beispiel 7*

*N-(1-Carbethoxy-3-cyclohexylpropyl)-S-alanyl-(2S, 3aS,7aS)-octahydroindol-2-carbonsäure-benzylester*

Hergestellt aus 2-R-Trifluormethansulfonyloxy-4--Cyclohexylbuttersäureethylester und S-Alanyl-(2S, 3aS,7aS)-octahydroindol-2-carbonsäurebenzylester nach dem in Beispiel 2 b angegebenen Verfahren.

| | | |
|---|---|---|
| $^1$H-NMR-Daten ($CDCl_3$) δ: | 7,3 | (s, 5H) |
| | 5,1 | (AB-System, 2H) |
| | 4,6-1,0 | (m, 30H) |
| | 1,3 | (t, 3H) ppm. |

*Beispiel 8*

*N-(1-Carbethoxy-3-phenyl-propyl)-S-alanyl-(2S, 3aS,6aS)-octahydrocyclopenta[b]pyrrol-2-carbonsäure-benzylester*

a) N-(2-R-Trifluormethansulfonyloxy-propionyl-(2S,3aS,6aS)-octahydrocyclopenta[b]pyrrol-2-carbonsäurebenzylester

Zu einer Lösung von 3,03 g (10 mmol) N-(2-R--Hydroxypropionyl)-(2S,3aS,6aS)-octahydrocyclopenta[b]pyrrol-2-carbonsäurebenzylester (hergestellt aus (2S,3aS,6aS)-octahydrocyclopenta[b]--pyrrol-2-carbonsäurebenzylester und Milchsäuretetrahydropyranyl in Gegenwart von Dicyclohexylcarbodiimid/1-Hydroxybenztriazol und nachfolgende sauer katalysierte Hydrolyse der Tetrahydropyranylgruppe) in 10 ml trockenem Dichlormethan tropft man unter Rühren bei 0°C eine Lösung von 0,8 g trockenem Pyridin und 3,25 g Trifluormethansulfonsäureanhydrid in 5 ml trockenem Dichlormethan.
Nach 15 Minuten bei 0°C wird der Niederschlag abfiltriert, das Filtrat eingeengt und an Kieselgel mit Cyclohexan/Essigester (4:1) als Laufmittel gereinigt.
Ausbeute: 76%.

b) N-(1-S-Carbethoxy-3-phenyl-propyl)-S-alanyl--(2S,3aS,6aS)-octahydrocyclopenta[b]pyrrol-2--carbonsäurebenzylester

3,3 g (7,6 mmol) der Verbindung aus Beispiel 8 a werden mit 1,57 g (7,6 mmol) S-Homophenylalaninethylester und 0,5 ml Triethylamin in 20 ml Dichlormethan 8 Stunden bei 25°C gerührt, eingeengt und an Kieselgel mit Cyclohexan/Essigester (1:1) als Laufmittel gereinigt.
m/e: 486.
Ausbeute: 67%.
Nach dem in Beispiel 1 und 2a sowie 8a angegebenen Verfahren weiterhin ausgehend von den entsprechenden 2-Hydroxycarbonsäureestern, die in der R- bzw. S- oder R,S-Form, bevorzugt der R-bzw. R,S-Form eingesetzt werden, beziehungsweise aus den entsprechenden Hydroxyacylaminosäureestern, die an dem die Hydroxygruppe tragenden C-Atomen die R- bzw. S- oder R,S-, vorzugsweise die R- oder R,S-Konfiguration aufweisen, die folgenden Trifluormethansulfonate hergestellt.

2-R,S-Trifluormethansulfonyloxy-5,5-dimethyl-hexansäureethylester
2-R-Trifluormethansulfonyloxy-5,5-dimethyl-hexansäureethylester
2-R,S-Trifluormethansulfonyloxy-4-cyclopentylbuttersäureethylester
2-R-Trifluormethansulfonyloxy-4-cyclopentylbuttersäureethylester
2-R,S-Trifluormethansulfonyloxy-4-(4-fluorphenyl)-buttersäureethylester
2-R-Trifluormethansulfonyloxy-4-(4-fluorphenyl)-buttersäureethylester
2-R,S-Trifluormethansulfonyloxy-4-(4-methoxyphenyl)-buttersäureethylester
2-R-Trifluormethansulfonyloxy-4-(4-methoxyphenyl)-buttersäureethylester
2-R,S-Trifluormethansulfonyloxy-4-(3,4-methoxyphenyl)-buttersäureethylester
2-R-Trifluormethansulfonyloxy-4-(3,4-methoxyphenyl)-buttersäureethylester
N-(2-R,S-Trifluormethansulfonyloxypropionyl)-S-prolinbenzylester
N-(2-R-Trifluormethansulfonyloxypropionyl)-S-prolinbenzylester
N-[2-R,S-Trifluormethansulfonyloxy-propionyl)-3-(4-ethoxyphenyl)-propionyl]-S-prolinbenzylester
N-(2-R,S-Trifluormethansulfonyloxy-propionyl)-1,2,3,4-tetrahydroisochinolin-3-S-carbonsäure-tert.-butylester
N-(2-R-Trifluormethansulfonyloxy-propionyl)-1,2,3,4-tetrahydroisochinolin-3-S-carbonsäure-tert.-butylester
N-(2-R-Trifluormethansulfonyloxy-propionyl)-decahydroisochinolin-3-S-carbonsäure-benzylester
N-(2-R-Trifluormethansulfonyloxy-propionyl)-(2S, 3aS,7aS)-octahydroindol-2-carbonsäure-benzylester
N-(2-R-Trifluormethansulfonyloxy-propionyl)-(2S, 3aR,7aS)-octahydroindol-2-carbonsäure-benzylester
N-(2-R-Trifluormethansulfonyloxy-propionyl)-(2S, 3aR,7aR)-octahydroindol-2-carbonsäure-benzylester
N-(2-R-Trifluormethansulfonyloxy-propionyl)-(2S, 3aS,7aR)-octahydroindol-2-carbonsäure-benzylester
N-(2-R-Trifluormethansulfonyloxy-propionyl)-(2S, 3aR,6aR)-octahydro-cyclopenta[b]pyrrol-2-carbonsäure-benzylester
N-(2-R-Trifluormethansulfonyloxy-propionyl)-(2S, 3aR,6aS)-octahydro-cyclopenta[b]pyrrol-2-carbonsäure-benzylester
N-(2-R-Trifluormethansulfonyloxy-propionyl)-2-azabicyclo[2.2.2]octan-3-S-carbonsäure-benzylester
N-(2-R-Trifluormethansulfonyloxy-propionyl)-exo-2-azabicyclo[2.2.1]heptan-3-S-carbonsäure-benzylester
N-(2-R-Trifluormethansulfonyloxy-propionyl)-endo-2-azabicyclo[2.2.1]heptan-3-S-carbonsäure-benzylester
N-(2-R-Trifluormethansulfonyloxy-propionyloxy)-2-azaspiro[4,5]decan-3-S-carbonsäure-benzylester
N-(2-R-Trifluormethansulfonyloxy-propionyl)-2-azaspiro[4,4]nonan-3-S-carbonsäure-benzylester
N-(2-R-Trifluormethansulfonyloxy-propionyl)-spiro-[bicyclo[2.2.2]octan-2,3'pyrrolidin]-5'-S-carbonsäure-benzylester
N-(2-R-Trifluormethansulfonyloxy-propionyl)-spiro-[bicyclo[2.2.1]heptan-2,3'pyrrolidin]-5'-S-carbonsäure-benzylester
N-(2-R-Trifluormethansulfonyloxy-propionyl)-2-azatricyclo[4.3.0.1$^{6,9}$]decan-3S-carbonsäure-benzylester
N-(2-R-Trifluormethansulfonyloxy-propionyl)-decahydrocyclohepta[b]pyrrol-2-S-carbonsäure-benzylester
N-(2-R-Trifluormethansulfonyloxy-propionyl)-cis-octahydroisoindol-1-S-carbonsdäure-benzylester
N-(2-R-Trifluormethansulfonyloxy-propionyl)-trans-octahydroisoindol-1-S-carbonsäure-benzylester
N-(2-R-Trifluormethansulfonyloxy-propionyl)-cis-octahydrocyclopenta[c]pyrrol-1-S-carbonsäure-benzylester
N-(2-R-Trifluormethansulfonyloxy-propionyl)-2,3,3a,4,5,7a-hexahydroindol-2-S-carbonsäure-tert.-butylester
N-(2-R-Trifluormethansulfonyloxy-propionyl)-thiazolidin-5-S-carbonsäure-tert.-butylester.

Die für die Herstellung der Trifluormethansulfonate erforderlichen 2-R,S-Hydroxycarbonsäureester werden aus den entsprechenden α-Ketoestern durch Reduktion mit Raney-Nickel und Wasserstoff erhalten. Ein weiteres Herstellungsverfahren besteht darin, dass die entsprechenden Cyanhydrine sauer verseift werden und die Hydroxycarbonsäure nach gängigen Veresterungsverfahren verestert werden.

Die 2-R- bzw. 2-R,S-Hydroxyacylaminosäureester werden aus den entsprechen den Aminosäureestern und geeignet geschützten Hydroxysäuren nach gängigen Amidbildungsmethoden und anschliessende Reduktion der α-Ketoacylaminosäureester mit Natriumhydrid erhalten.

Die Racematspaltung der racemischen 2-Hydroxycarbonsäuren geschieht entweder über diastereomere Salzbildung mit optisch aktiven Aminen oder Aminosäureestern und fraktionierte Kristallisation oder durch Veresterung mit optisch aktiven Alkoholen wie z.B. Mentol, die säulenchromatographisch oder durch fraktionierte Kristallisation getrennt werden. Die Veresterung zu den diastereomeren 2-Hydroxycarbonsäureestern geschieht nach üblichen Veresterungsmethoden.

Nach den in Beispiel 1 und 2b beschriebenen Verfahren werden die oben beschriebenen 2-Trifluormethansulfonyloxycarbonsäureester mit den entsprechenden Dipeptidestern oder nach dem in Beispiel 8 b beschriebenen Verfahren die obenbeschriebenen 2-Trifluormethansulfonyloxy-acylaminosäureester mit den entsprechenden Aminosäureestern zu den folgenden Verbindungen umgesetzt:

N-(1-S-Carbethoxy-3-phenyl-propyl)-S-alanyl-S-prolin-benzylester

N-(1-R-Carbethoxy-3-phenyl-propyl)-S-alanyl-S--prolin-benzylester
N-(1-R,S-Carbethoxy-3-phenyl-propyl)-S-alanyl-S--prolin-benzylester
N-(1-S-Carbethoxy-3-cyclohexyl-propyl)-S-alanyl--S-prolin-benzylester
N-(1-R-Carbethoxy-3-cyclohexyl-propyl)-S-alanyl--S-prolin-benzylester
N-(1-S-Carbethoxy-3-phenyl-propyl)-$N_\varepsilon$-benzyloxycarbonyl-S-lysyl-S-prolin-tert. butylester
N-(1-S-Carbethoxy-3-phenyl-propyl)-S-tyrosyl-S--prolin-benzylester
N-(1-S-Carbethoxy-3-phenyl-propyl)-O-methyl-S--tyrosyl-S-prolin-benzylester
N-(1-S-Carbethoxy-3-phenyl-propyl)-S-alanyl-S-pipecolsäure-benzylester
N-(1-S-Carbethoxy-3-cyclohexyl-propyl)-S-alanyl--S-pipecolsäurebenzylester
N-(1-S-Carbethoxy-3-cyclohexyl-propyl)-S-alanyl--S-1,2,3,4-tetrahydroisochinolin-3-carbonsäure--tert. butylester
N-(1-S-Carbethoxy-3-phenyl-propyl)-$N_\varepsilon$-benzyloxycarbonyl-S-lysyl-S-1,2,3,4-tetrahydroisochinolin-3--carbonsäure-tert. butylester
N-(1-S-Carbethoxy-3-phenyl-propyl)-O-ethyl-S--tyrosyl-S-1,2,3,4-tetrahydroisochinolin-3-carbonsäure-tert. butylester
N-(1-S-Carbethoxy-3-phenyl-propyl)-S-alanyl-3S--decahydroisochinolin-3-carbonsäure-benzylester
N-(1-S-Carbethoxy-3-phenyl-propyl)-S-alanyl-(2S, 3aS,7aS)-octahydroindol-2-carbonsäure-benzylester
N-(1-S-Carbethoxy-3-phenyl-propyl)-$N_\varepsilon$-benzyloxycarbonyl-S-lysyl-(2S,3aS,7aS)-octahydroindol-2--carbonsäure-tert. butylester
N-(1-S-Carbethoxy-3-cyclohexylpropyl)-$N_\varepsilon$-benzyloxycarbonyl-S-lysyl-(2S,3aS,7aS)-octahydroindol--2-carbonsäure-tert. butylester
N-(1-S-Carbethoxy-3-phenyl-propyl)-O-methyl-S--tyrosyl-(2S,3aS,7aS)-octahydroindol-2-carbonsäure-benzylester
N-(1-S-Carbethoxy-4,4-dimethylpentyl)-S-alanyl--(2S,3aS,7aS)-octahydroindol-2-carbonsäurebenzylester
N-[1-S-Carbethoxy-3-(4-fluorphenyl)propyl]-S--alanyl-(2S,3aS,7aS)-octahydroindol-2-carbonsäure-benzylester
N-[1-S-Carbethoxy-3-(4-methoxyphenyl)-propyl]-S--alanyl-(2S,3aS,7aS)-octahydroindol-2-carbonsäure-benzylester
N-[1-S-Carbethoxy-3-(3,4-dimethoxyphenyl)-propyl]-S-alanyl-(2S,3aS,7aS)-octahydroindol-2-carbonsäure-benzylester
N-[1-S-Carbethoxy-3-(cyclopentylpropyl]-S-alanyl--(2S,3aS,7aS)-octahydroindol-2-carbonsäure-benzylester
N-(1-S-Carbethoxy-3-phenyl-propyl)-S-alanyl-(2S, 3aS,7aS)-octahydroindol-2-carbonsäure-benzylester
N-(1-S-Carbethoxy-3-cyclohexyl-propyl)-S-alanyl--(2S,3aS,7aS)-octahydroindol-2-carbonsäure-benzylester
N-(1-S-Carbethoxy-3-phenyl-propyl)-$N_\varepsilon$-benzyloxycarbonyl-S-lysyl-(2S,3aR,7aS)-octahydroindol-2--carbonsäure-tert. butylester
N-(1-S-Carbethoxy-3-cyclohexyl-propyl)-$N_\varepsilon$-benzyloxycarbonyl-S-lysyl-(2S,3aR,7aS)-octahydroindol--2-carbonsäure-benzylester
N-(1-S-Carbethoxy-3-phenyl-propyl)-O-ethyl-S--tyrosyl-(2S,3aR,7aS)-octahydroindol-2-carbonsäure-benzylester
N-(1-S-Carbethoxy-3-phenyl-propyl)-S-alanyl-(2S, 3aR,7aR)-octahydroindol-2-carbonsäure-benzylester
N-(1-S-Carbethoxy-3-phenyl-propyl)-$N_\varepsilon$-benzyloxycarbonyl-S-lysyl-(2S,3aR,7aR)-octahydroindol-2--carbonsäure-tert. butylester
N-(1-S-Carbethoxy-3-cyclohexyl-propyl)-S-alanyl--(2S,3aR,7aR)-octahydroindol-2-carbonsäure-benzylester
N-(1-S-Carbethoxy-3-phenyl-propyl)-S-alanyl-(2S, 3aS,7aR)-octahydroindol-2-carbonsäure-benzylester
N-(1-S-Carbethoxy-3-phenyl-propyl)-S-alanyl-(2S, 3aS,6aS)-octahydrocyclopenta[b]pyrrol-2-carbonsäure-benzylester
N-(1-S-Carbethoxy-3-cyclohexyl-propyl)-$N_\varepsilon$-benzyloxycarbonyl-S-lysyl-(2S,3aS,6aS)-octahydrocyclopenta[b]pyrrol-2-carbonsäure tert. butylester
N-(1-S-Carbethoxy-3-phenyl-propyl)-O-methyl-S--tyrosyl-(2S,3aS,6aS)-octahydrocyclopenta[b]-pyrrol-2-carbonsäure-benzylester
N-(1-S-Carbethoxy-3-phenyl-propyl)-O-ethyl-S-tyrosyl-(2S,3aS,6aS)-octahydrocyclopenta[b]-pyrrol-2-carbonsäure-benzylester
N-(1-S-Carbethoxy-3-[4-fluor-phenyl]-propyl)-S--alanyl-(2S,3aS,6aS)-octahydrocyclopenta[b]pyrrol-2-carbonsäure-benzylester
N-(1-S-Carbethoxy-3-[4-methoxyphenyl]-S-alanyl--(2S,3aS,6aS)-octahydrocyclopenta[b]pyrrol-2-carbonsäure-benzylester
N-(1-S-Carbethoxy-3-cyclopentylpropyl)-S-alanyl--(2S,3aS,6aS)-octahydrocyclopenta[b]pyrrol-2-carbonsäure-benzylester
N-(1-S-Carbethoxy-3-phenyl-propyl)-S-alanyl-(2S, 3aR,6aR)-octahydrocyclopenta[b]pyrrol-2-carbonsäure-benzylester
N-(1-S-Carbethoxy-3-cyclohexylpropyl)-S-alanyl--(2S,3aR,6aR)-octahydrocyclopenta[b]pyrrol-2-carbonsäure-benzylester
N-(1-S-Carbethoxy-3-phenyl-propyl)-$N_\varepsilon$-benzyloxycarbonyl-S-lysyl-(2S,3aR,6aR)-octahydrocyclopenta[b]pyrrol-2-carbonsäure-benzylester
N-(1-S-Carbethoxy-3-phenyl-propyl)-S-alanyl-(2S, 3aR,6aS)-octahydrocyclopenta[b]pyrrol-2-carbonsäure-benzylester
N-(1-S-Carbethoxy-3-cyclohexylpropyl)-S-alanyl--(2S,3aR,6aS)-octahydrocyclopenta[b]pyrrol-2-carbonsäure-benzylester
N-(1-S-Carbethoxy-3-phenyl-propyl)-$N_\varepsilon$-benzyloxycarbonyl-S-lysyl-(2S,3aR,6aS)-octahydrocyclopenta[b]pyrrol-2-carbonsäure-tert. butylester
N-(1-S-Carbethoxy-3-cyclohexylpropyl)-$N_\varepsilon$-benzyloxycarbonyl-S-lysyl-(2S,3aR,6aS)-octahydrocyclopenta[b]pyrrol-2-carbonsäure-tert. butylester
N-(1-S-Carbethoxy-3-phenyl-propyl)-O-ethyl-S--tyrosyl-(2S,3aR,6aS)-octahydrocyclopenta[b]pyrrol-2-carbonsäure-benzylester
N-(1-S-Carbethoxy-3-cyclopentyl-propyl)-S-alanyl--2-alanyl-(2S,3aR,6aS)-octahydrocyclopenta[b]-pyrrol-2-carbonsäure-benzylester
N-(1-S-Carbethoxy-3-phenyl-propyl)-S-alanyl-S-2-azabicyclo[2.2.2]octan-3-carbonsäure-benzylester
N-(1-S-Carbethoxy-3-phenyl-propyl)-O-ethyl-S-tyrosyl-S-2-azabicyclo[2.2.2]-octan-3-carbonsäure-benzylester
N-(1-S-Carbethoxy-3-cyclohexyl-propyl)-S-alanyl-S-azabicyclo[2.2.2]-octan-3-carbonsäure-benzylester
N-(1-S-Carbethoxy-3-cyclohexyl-propyl)-$N_ε$-benzyloxycarbonyl-S-2-azabicyclo[2.2.2]-octan-3-carbonsäure-tert. butylester
N-(1-S-Carbethoxy-3-phenyl-propyl)-$N_ε$-benzyloxycarbonyl-S-lysyl-S-2-azabicyclo[2.2.2]-octan-3-carbonsäure-tert. butylester
N-(1-S-Carbethoxy-3-cyclopentylpropyl)-S-alanyl-S-2-azabicyclo[2.2.2]-octan-3-carbonsäure-benzylester
N-(1-S-Carbethoxy-3-phenyl-propyl)-S-alanyl-3S-exo-2-azabicyclo[2.2.1]-heptan-3-carbonsäure-benzylester
N-(1-S-Carbethoxy-3-cyclohexylpropyl)-S-alanyl-3S-exo-2-azabicyclo[2.2.1]-heptan-3-carbonsäure-benzylester
N-(1-S-Carbethoxy-3-phenyl-propyl)-$N_ε$-benzyloxycarbonyl-S-lysyl-3S-lysyl-3S-exo-2-azabicyclo-[2.2.1]-heptan-3-carbonsäure-tert. butylester
N-(1-S-Carbethoxy-3-phenyl-propyl)-S-alanyl-3S-endo-2-azabicyclo[2.2.1]-heptan-3-carbonsäure-benzylester
N-(1-S-Carbethoxy-3-cyclohexylpropyl)-S-alanyl-3S-endo-2-azabicyclo[2.2.1]-heptan-3-carbonsäure-benzylester
N-(1-S-Carbethoxy-3-phenyl-propyl)-$N_ε$-benzyloxycarbonyl-S-lysyl-3S-endo-2-azabicyclo[2.2.1]-heptan-3-carbonsäure-tert. butylester
N-(1-S-Carbethoxy-3-phenyl-propyl)-O-ethyl-S-tyrosyl-3S-endo-2-azabicyclo[2.2.1]-heptan-3-carbonsäure-butylester
N-(1-S-Carbethoxy-3-phenyl-propyl)-S-alanyl-2-azaspiro[4,5]decan-3-S-carbonsäure-benzylester
N-(1-S-Carbethoxy-3-phenyl-propyl)-O-ethyl-S-tyrosyl-2-azaspiro[4,5]decan-3-S-carbonsäure-benzylester
N-(1-S-Carbethoxy-3-phenyl-propyl)-$N_ε$-benzyloxycarbonyl-S-lysyl-2-azaspiro[4,5]decan-3-S-carbonsäure-tert. butylester
N-(1-S-Carbethoxy-3-cyclohexylpropyl)-S-alanyl-2-azaspiro[4,5]decan-3-S-carbonsäure-benzylester
N-(1-S-Carbethoxy-3-cyclohexylpropyl)-$N_ε$-benzyloxycarbonyl-S-lysyl-2-azaspiro[4,5]decan-3-S-carbonsäure-tert. butylester
N-(1-S-Carbethoxy-3-phenyl-propyl)-S-alanyl-2-azaspiro[4,4]nonan-3-S-carbonsäure-benzylester
N-(1-S-Carbethoxy-3-phenyl-propyl)-O-ethyl-S-tyrosyl-2-azaspiro[4,4]nonan-3-S-carbonsäure-benzylester
N-(1-S-Carbethoxy-3-phenyl-propyl)-S-alanylspiro-[bicyclo[2.2.1]heptan-2,3'-pyrrolidin]-5'-S-carbonsäure-benzylester
N-(1-S-Carbethoxy-3-phenyl-propyl)-O-ethyl-S-tyrosyl spiro[bicyclo[2.2.1]heptan-2,3'-pyrrolidin]-5'-S-carbonsäure-benzylester
N-(1-S-Carbethoxy-3-phenyl-propyl)-$N_ε$-benzyloxycarbonyl-S-lysyl spiro[bicyclo[2.2.1]heptan-2,3'-pyrrolidin]-5'-S-carbonsäure-tert. butylester
N-(1-S-Carbethoxy-3-cyclohexyl-propyl)-S-alanylspiro[bicyclo[2.2.1]heptan-2,3'-pyrrolidin]-5'-carbonsäure-benzylester
N-(1-S-Carbethoxy-3-cyclohexyl-propyl)-$N_ε$-tert.-butoxycarbonyl-S-lysylspiro[bicyclo[2.2.1]heptan-2,3'-pyrrolidin]-5'-S-carbonsäure-benzylester
N-(1-S-Carbethoxy-3-phenyl-propyl)-S-alanylspiro-[bicyclo[2.2.2]octan-2,3'-pyrrolidin]-5'-S-carbonsäure-benzylester
N-(1-S-Carbethoxy-3-phenyl-propyl)-O-ethyl-S-tyrosylspiro[bicyclo[2.2.2]octan-2,3'-pyrrolidin]-5'-carbonsäure-benzylester
N-(1-S-Carbethoxy-3-phenyl-propyl)-$N_ε$-tert. butoxycarbonyl-S-lysylspiro[bicyclo[2.2.2]octan-2,3'-pyrrolidin]-5'-S-carbonsäure-benzylester
N-(1-S-Carbethoxy-3-cyclohexyl-propyl)-S-alanylspiro[bicyclo[2.2.2]octan-2,3'-pyrrolidin]-5'-S-carbonsäure-benzylester
N-(1-S-Carbethoxy-3-phenyl-propyl)-S-alanyl-azatricyclo[4,3,0,$1^{6,9}$]decan-3-S-carbonsäure-benzylester
N-(1-S-Carbethoxy-3-phenyl-propyl)-O-ethyl-S-tyrosyl-2-azatricyclo[4,3,0,$1^{6,9}$]decan-3-S-carbonsäure-benzylester
N-(1-S-Carbethoxy-3-phenyl-propyl)-$N_ε$-benzyloxycarbonyl-S-lysyl-2-azatricyclo[4,3,0,$1^{6,9}$]decan-3-S-carbonsäure-tert. butylester
N-(1-S-Carbethoxy-3-cyclohexyl-propyl)-S-alanyl-2-azatricyclo[4,3,0,$1^{6,9}$]decan-3-S-carbonsäure-benzylester
N-(1-S-Carbethoxy-3-phenyl-propyl)-$N_ε$-benzyloxycarbonyl-S-lysyl-2-azatricyclo[4,3,0,$1^{6,9}$]decan-3-S-carbonsäure-tert. butylester
N-(1-S-Carbethoxy-3-phenyl-propyl)-S-alanyl decahydrocyclohepta[b]pyrrol-2-S-carbonsäure-benzylester
N-(1-S-Carbethoxy-3-phenyl-propyl)-O-ethyl-S-tyrosyl-decahydrocyclohepta[b]pyrrol-2-S-carbonsäure-benzylester
N-(1-S-Carbethoxy-3-phenyl-propyl)-$n_ε$-benzyloxycarbonyl-S-lysyl-decahydrocyclohepta[b]pyrrol-2-S-carbonsäure-tert. butylester
N-(1-S-Carbethoxy-3-cyclohexyl-propyl)-S-alanyl-decahydrocyclohepta[b]pyrrol-2-S-carbonsäure-benzylester
N-(1-S-Carbethoxy-3-cyclohexyl-propyl)-$N_ε$-tert.-butoxycarbonyl-S-lysyl decahydrocyclohepta[b]-pyrrol-2-S-carbonsäure-tert.-butylester
N-(1-S-Carbethoxy-3-phenyl-propyl)-S-alanyl-trans-octahydroisoindol-1-S-carbonsäure-benzylester
N-(1-S-Carbethoxy-3-phenyl-propyl)-S-alanyl-cis-octahydroisoindol-1-S-carbonsäure-benzylester
N-(1-S-Carbethoxy-3-cyclohexyl-propyl)-S-alanyl-trans-octahydroisoindol-1-S-carbonsäure-benzylester
N-(1-S-Carbethoxy-3-cyclohexyl-propyl)-S-alanyl-cis-octahydroisoindol-1-S-carbonsäure-benzylester
N-(1-S-Carbethoxy-3-phenyl-propyl)-S-alanyl-cis-octahydrocyclopenta[c]pyrrol-1-S-carbonsäure-benzylester

N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-cyclohexyl-propyl)-$N_\varepsilon$-tert.-butoxycarbonyl-S-lysyl-cis-octahydrocyclopenta[c]pyrrol-1-S-carbonsäure-benzylester
N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-phenyl-propyl)-S-alanyl-2,3,3a,4,5,7a-hexahydroindol-2-S-carbonsäure-tert.-butylester
N-(1-S-Carbethoxy-3-phenyl-propyl)-O-ethyl-S-tyrosyl-2,3,3a,4,5,7a-hexahydroindol-2-S-carbonsäure-tert.-butylester
N-(1-S-Carbethoxy-3-phenyl-propyl)-S-alanyl-thiazolidin-5-S-carbonsäure-tert.-butylester
N-(1-S-Carbethoxy-3-cyclohexyl-propyl)-S-alanyl-thiazolidin-5-S-carbonsäure-tert.-butylester
N-(1-S-Carbethoxy-3-phenyl-propyl)-$N_\varepsilon$-benzyloxycarbonyl-lysyl-thiazolidin-5-S-carbonsäure-tert.-butylester.

Bei Anwendung der Edukte, die am Trifluormethan-Sulfonat tragenden C-Atom racemisch sind, erhält man die N-Alkyldipeptide mit R,S-Konfiguration in N-Alkylteil.

**Patentansprüche für die Vertragsstaaten:**
BE, CH, DE, FR, GB, IT, LI, LU, NL, SE

1. Verfahren zur Herstellung von Verbindungen der Formel I

```
        *         *       *
R3OOC-CH-N - C -CH-NH-CH-(CH2)n-R        (I)
      |   |  ||  |      |
      R4  R5 O   R1     COOR2
```

in welcher
n 1 oder 2 ist
R Wasserstoff,
einen gegebenenfalls substituierten aliphatischen Rest mit 1 bis 8 C-Atomen,
einen gegebenenfalls substituierten cycloaliphatischen Rest mit 3-9 C-Atomen,
einen gegebenenfalls substituierten aromatischen Rest mit 6-12 C-Atomen,
einen gegebenenfalls substituierten araliphatischen Rest mit 7-14 C-Atomen,
einen gegebenenfalls substituierten cycloaliphatisch-aliphatischen Rest mit 7-14 C-Atomen,
einen Rest $OR^a$ oder $SR^a$, worin
$R^a$ für einen gegebenenfalls substituierten aliphatischen Rest mit 1-4 C-Atomen, für einen gegebenenfalls substituierten aromatischen Rest mit 6-12 C-Atomen oder einen gegebenenfalls substituierten heteroaromatischen Rest mit 5-12 Ringatomen steht,
$R^1$ Wasserstoff,
einen gegebenenfalls substituierten aliphatischen Rest mit 1 bis 6 C-Atomen,
einen gegebenenfalls substituierten cycloaliphatischen Rest mit 3-9 C-Atomen,
einen gegebenenfalls substituierten cycloaliphatisch-aliphatischen Rest mit 4-13 C-Atomen,
einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-12 Ringatomen oder
die Seitenkette einer gegebenenfalls geschützten natürlich vorkommenden α-Aminosäure bedeuten,
$R^2$ und $R^3$ gleich oder verschieden sind und Wasserstoff,
einen gegebenenfalls substituierten aliphatischen Rest mit 1-6 C-Atomen,
einen gegebenenfalls substituierten cycloaliphatischen Rest mit 3-9 C-Atomen,
einen gegebenenfalls substituierten aromatischen Rest mit 6-12 C-Atomen,
einen gegebenenfalls substituierten araliphatischen Rest mit 7-16 C-Atomen bedeuten und
$R^4$ und $R^5$ zusammen mit den sie tragenden Atomen ein heterocyclisches mono-, bi-, oder tricyclisches Ringsystem mit 3 bis 15 C-Atomen bilden,

dadurch gekennzeichnet, dass man eine Verbindung der Formel II

```
              OSO2CF3
            * /
R(CH2)n-CH                    (II)
            \
              COOR2
```

in welcher n, R und $R^2$ wie oben definiert sind, mit einer Verbindung der Formel IV

```
        *a        *b
R3OOC-CH-N - C -CH-NH2         (IV)
      |   |  ||  |
      R4  R5 O   R1
```

in welcher $R^1$, $R^3$, $R^4$ und $R^5$ wie oben definiert sind, oder eine Verbindung der Formel III

```
        *a        *b
R3OOC-CH-N - C -CH-OSO2CF3     (III)
      |   |  ||  |
      R4  R5 O   R1
```

in welcher $R^1$, $R^3$, $R^4$ und $R^5$ wie oben definiert sind, mit einer Verbindung der Formel V

```
           *
R-(CH2)n-CH-NH2                (V)
           |
           COOR2
```

in welcher n, R und $R^2$ wie oben definiert sind, in Gegenwart von mindestens einem Äquivalent einer Base, die nicht mit Verbindungen der Formeln II oder III abreagieren kann, umsetzt, gegebenenfalls Estergruppen hydrolytisch oder hydrogenolytisch abspaltet und gegebenenfalls freie Carboxygruppen in an sich bekannter Weise verestert.

2. Verfahren gemäss Anspruch 1, dadurch gekennzeichnet, dass Verbindungen der Formel I hergestellt werden, worin $R^4$ und $R^5$ zusammen mit dem sie tragenden Atomen für ein gegebenenfalls substituiertes System aus der Reihe Pyrrolidin, Piperidin, Tetrahydroisochinolin, Decahydroisochinolin, Octahydroindol, Octahydrocyclopenta[b]pyrrol, 2-Aza-bicyclo[2.2.2]octan, 2-Azabicyclo-[2.2.2]-heptan, 2-Azaspiro[4,5]decan, 2-Azaspiro-[4,4]nonan, Spiro[(bicyclo[2.2.1]heptan)-2,3-pyrrolidin], Spiro[(bicyclo[2.2.2]octan)-2,3-pyrrolidin], 2-Azatricyclo[4,3,0,$1^{6,9}$]decan, Decahydrocyclohepta[b]pyrrol, Octahydroisoindol, Octahydrocyclopenta[c]pyrrol, 2,3,3a,4,5,7a-Hexahydroindol und Tetrahydrothiazol stehen.

3. Verfahren gemäss einem der Ansprüche 1 oder 2, dadurch gekennzeichnet, dass Verbindungen der Formel I hergestellt werden, in welcher

n 1 oder 2 ist

R Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen ,
das wie oben bei Aryl beschrieben substituiert sein kann,
mono bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen, das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die Seitenkette einer gegebenenfalls geschützten natürlich vorkommenden α-Aminosäure $R^1$-CH($NH_2$-COOH bedeuten

$R^2$ und $R^3$ gleich oder verschieden sind und
Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl
Aryl mit 6-12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und

$R^4$ und $R^5$ die oben angegebene Bedeutung haben.

4. Verfahren gemäss einem der Ansprüche 1 bis 3, dadurch gekennzeichnet ist, dass Verbindungen der Formel I herstellt werden, in welcher

n 1 oder 2 ist

R ($C_1$- bis $C_6$)-Alkyl, ($C_2$ bis $C_6$)-Alkenyl, ($C_3$ bis $C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$ bis $C_{12}$)-Aryl-($C_1$-$C_4$)-alkoxycarbonylamino-($C_1$-$C_4$)-alkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylendioxy mono-, di- oder trisubstituiert sein kann, oder 3-Indolyl

$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis

$C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure,

$R^2$ und $R^3$ gleiche oder verschiedene Reste Wasserstoff,
($C_1$ bis $C_6$)-Alkyl, ($C_2$ bis $C_6$)-Alkenyl oder
($C_6$ bis $C_{12}$)-Aryl-($C_1$ bis $C_4$)-alkyl Bedeuten und

$R^4$ und $R^5$ die oben angegebene Bedeutung haben.

5. Verfahren gemäss einem der Ansprüche 1 bis 4, dadurch gekennzeichnet, dass Verbindungen der Formel I hergestellt werden in welcher

n 1 oder 2 ist,

R Methyl, Ethyl, Cyclohexyl, tert.-Butoxycarbonylamino-($C_1$-$C_4$)-alkyl oder Phenyl, das durch Phenyl, ($C_1$ bis $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $c_4$)alkylamino, Nitro und/oder Methylendioxy mono- oder disubstituiert oder im Falle von Methoxy, trisubstituiert sein kann,

$R^1$ Wasserstoff, ($C_1$ bis $C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl

$R^2$ und $R^3$ gleiche oder verschiedene Reste Wasserstoff, ($C_1$ bis $C_4$)-Alkyl oder Benzyl bedeuten und

$R^4$ und $R^5$ die oben angegebene Bedeutung haben.

6. Verbindungen der Formel III, in welcher

$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon
1 bis 2 Ringatome Scvhwefel- oder Sauerstoffatome und/oder 1 bis 4 Ringatome Stickstoffatome darstellen, oder eine Seitenkette einer natürlich vorkommenden, gegebenenfalls geschützten α-Aminosäure,

$R^3$ Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl bedeuten und

$R^4$ und $R^5$ die bei Verbindungen der Formel I angegebene Bedeutung haben.

7. Verbindung der Formel III, gemäss Anspruch 6, in welcher

$R^1$ Wasserstoff, ($C_1$ bis $C_3$)-Alkyl oder ($C_2$ bis $C_3$)-Alkenyl, die gegebenenfalls geschützte Seitenkette von Lysin, Benzyl, 4-Methoxybenzyl, 4-Ethoxybenzyl, Phenethyl, 4-Aminobutyl oder Benzoylmethyl

$R^3$ Wasserstoff, ($C_1$ bis $C_4$)-Alkyl oder Benzyl bedeuten und

$R^4$ und $R^5$ die bei Verbindungen der Formel I angegebene Bedeutung haben.

8. Verbindung der Formel III gemäss Anspruch 6, in welcher

$R^1$ Methyl, Ethyl, Phenyl, die gegebenenfalls acylierte Seitenkette von Lysin, Benzyl oder die O-($C_1$-$C_6$)-alkylierte Seitenkette von Tyrosin,

$R^3$ Wasserstoff, methyl, Ethyl, tert. Butyl oder Benzyl bedeuten und

$R^4$ und $R^5$ die bei Verbindungen der Formel I angegebene Bedeutung haben.

9. Verfahren zur Herstellung von Verbindungen der Formel III, in welcher $R^1$, $R^3$, $R^4$ und $R^5$ die obengenannten Bedeutungen haben, dadurch gekennzeichnet, dass man α-Hydroxycarbonsäurederivate der Formel VII

```
       *a        *b
R3OOC-CH- N -C - CH-OH          (VII)
       |    |  |   |
       R4   R5 O   R1
```

in welcher $R^1$, $R^3$, $R^4$ und $R^5$ die obengenannten Bedeutungen haben, mit einem Trifluormethansulfonierungsmittel gegebenenfalls in Gegenwart einer Base umsetzt.

**Patentansprüche für den Vertragsstaat:** AT

1. Verfahren zur Herstellung von Verbindungen der Formel I

```
       *         *       *
R3OOC-CH-N - C -CH-NH-CH-(CH2)n-R       (I)
       |   |  ||  |      |
       R4  R5 O   R1     COOR2
```

in welcher

n 1 oder 2 ist,

R Wasserstoff,
einen gegebenenfalls substituierten aliphatischen Rest mit 1 bis 8 C-Atomen,
einen gegebenenfalls substituierten cycloaliphatischen Rest mit 3-9 C-Atomen,
einen gegebenenfalls substituierten aromatischen Rest mit 6-12 C-Atomen,
einen gegebenenfalls substituierten araliphatischen Rest mit 7-14 C-Atomen,
einen gegebenenfalls substituierten cycloaliphatisch-aliphatischen Rest mit 7-14 C-Atomen,
einen Rest $OR^a$ oder $SR^a$, worin

$R^a$ für einen gegebenenfalls substituierten aliphatischen Rest mit 1-4 C-Atomen, für einen gegebenenfalls substituierten aromatischen Rest mit 6-12 C-Atomen oder einen gegebenenfalls substituierten heteroaromatischen Rest mit 5-12 Ringatomen steht,

$R^1$ Wasserstoff,
einen gegebenenfalls substituierten aliphatischen Rest mit 1 bis 6 C-Atomen,
einen gegebenenfalls substituierten cycloaliphatischen Rest mit 3-9 C-Atomen,
einen gegebenenfalls substituierten cycloaliphatisch-aliphatischen Rest mit 4-13 C-Atomen,
einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-12 Ringatomen oder
die Seitenkette einer gegebenenfalls geschützten natürlich vorkommenden α-Aminosäure bedeuten,

$R^2$ und $R^3$ gleich oder verschiedene sind und Wasserstoff,
einen gegebenenfalls substituierten aliphatischen Rest mit 1-6 C-Atomen,
einen gegebenenfalls substituierten cycloaliphatischen Rest mit 3-9 C-Atomen,
einen gegebenenfalls substituierten aromatischen Rest mit 6-12 C-Atomen,
einen gegebenenfalls substituierten araliphatischen Rest mit 7-16 C-Atomen bedeuten und

$R^4$ und $R^5$ zusammen mit den sie tragenden Atomen ein heterocyclisches mono-, bi-, oder tricyclisches Ringsystem mit 3 bis 15 C-Atomen bilden,

dadurch gekennzeichnet, dass man eine Verbindung der Formel II

```
           OSO2CF3
        * /
R(CH2)n-CH                         (II)
          \
           COOR2
```

in welcher n, R und $R^2$ wie oben definiert sind, mit einer Verbindung der Formel IV

```
      *a       *b
R3OOC-CH-N - C -CH-NH2             (IV)
      |  |   ||  |
      R4 R5  O   R1
```

in welcher $R^1$, $R^3$, $R^4$ und $R^5$ wie oben definiert sind, oder eine Verbindung der Formel III

```
      *a       *b
R3OOC-CH-N - C -CH-OSO2CF3         (III)
      |  |   ||  |
      R4 R5  O   R1
```

in welcher $R^1$, $R^3$, $R^4$ und $R^5$ wie oben definiert sind, mit einer Verbindung der Formel V

```
          *
R-(CH2)n-CH-NH2                    (V)
          |
          COOR2
```

in welcher n, R und $R^2$ wie oben definiert sind, in Gegenwart von mindestens einem Äquivalent einer Base, die nicht mit Verbindungen der Formeln II oder III abreagieren kann, umsetzt, gegebenenfalls Estergruppen hydrolytisch oder hydrogenolytisch abspaltet und gegebenenfalls freie Carboxygruppen in an sich bekannter Weise verestert.

2. Verfahren gemäss Anspruch 1, dadurch gekennzeichnet, dass Verbindungen der Formel I hergestellt werden, worin $R^4$ und $R^5$ zusammen mit dem sie tragenden Atomen für ein gegebenenfalls substituiertes System aus der Reihe Pyrrolidin, Piperidin, Tetrahydroisochinolin, Decahydroisochinolin, Octahydroindol, Octahydrocyclopenta[b]pyrrol, 2-Azabicyclo[2.2.2]octan, 2-Azabicyclo[2.2.1]-heptan, 2-Azaspiro[4,5]decan, 2-Azaspiro[4,4]-nonan, Spiro[(bicyclo]heptan)-2,3-pyrrolidin], Spiro[(bicyclo[2.2.2]octan)-2,3-pyrrolidin], 2-Azatricyclo[4,3,0,$1^{6,9}$]decan, Decahydrocyclohepta[b]-pyrrol, Octahydroisoindol, Octahydrocyclopenta[c]pyrrol, 2,3,3a,4,5,7a-Hexahydroindol und Tetrahydrothiazol stehen.

3. Verfahren gemäss einem der Ansprüche 1 oder 2, dadurch gekennzeichnet, dass Verbindungen der Formel I hergestellt werden, in welcher

n 1 oder 2 ist,

R Wasserstoff,
Alkyl mit 1-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,

Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben substituiert sein kann,
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-akoxy,
das im Arylteil wie oben bei Aryl beschrieben substi-
tuiert sein kann,

$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6-12 C-Atomen, das wie oben bei R beschrieben substituiert sein kann,
$(C_6-C_{12})$-Aryl-$(C_1-C_4)$-alkyl oder $(C_7-C_{13})$-Aroyl-$(C_1$ oder $C_2)$alkyl
die beide wie das vorstehende Aryl substituiert sein können
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die Seitenkette einer gegebenenfalls geschützten natürlich vorkommenden α-Aminosäure $R^1$-$CH(NH_2)$-$COOH$ bedeuten,

$R^2$ und $R^3$ gleich oder verschieden sind und Wasserstoff,
Alkyl mit 1-6 C-Atomen,
Alkenyl mit 2-6 C-Atomen,
Di-$(C_1-C_4)$-alkylamino-$(C_1-C_4)$-alkyl,
$(C_1-C_5)$-Alkanoylamino-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl Aryl mit 6-12 C-Atomen,
$(C_6-C_{12})$-Aryl-$(C_1-C_4)$-alkyl,
$(C_3-C_9)$-Cycloalkyl oder
$(C_3-C_9)$-Cycloalkyl-$(C_1-C_4)$-alkyl
bedeuten und

$R^4$ und $R^5$ die oben angegebene Bedeutung haben.

4. Verfahren gemäss einem der Ansprüche 1 bis 3, dadurch gekennzeichnet ist, dass Verbindungen der Formel I hergestellt werden, in welcher

n 1 oder 2 ist,

R $(C_1$ bis $C_6)$-Alkyl, $C_2$ bis $C_6)$-Alkenyl, $(C_3$ bis $C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6$ bis $C_{12})$-Aryl-$(C_1-C_4)$-alkoxycarbonylamino-$(C_1-C_4)$-alkyl, $(C_6$ bis $C_{12})$-Aryl, das durch $(C_1$ bis $C_4)$-Alkyl, $(C_1$ bis $C_4)$-Alkoxy, Hydroxy, Halogen, Nitro, Amino, $(C_1$ bis $C_4)$-Alkylamino, Di-$(C_1$ bis $C_4)$alkyl-amino und/oder Methylendioxy mono-, di- oder trisubstituiert sein kann, oder 3-Indolyl

$R^1$ Wasserstoff oder $(C_1$ bis $C_6)$-Alkyl, das gegebenenfalls durch Amino, $(C_1$ bis $C_6)$-Acylamino oder Benzoylamino substituiert sein kann, $(C_2$ bis $C_6)$-Alkenyl, $(C_3$ bis $C_9)$-Cycloalkyl, $(C_5$ bis $C_9)$-Cycloalkenyl, $(C_3$ bis $C_7)$-Cycloalkyl-$(C_1$ bis $C_4)$-alkyl, $(C_6$ bis $C_{12})$-Aryl oder teilhydriertes Aryl, das jeweils durch $(C_1$ bis $C_4)$-Alkyl, $(C_1$ oder $C_2)$-Alkoxy oder Halogen substituiert sein kann, $(C_6-C_{12}$ )-Aryl-$(C_1$ bis $C_4)$-alkyl oder $(C_7-C_{13})$-Aroyl-$(C_1-C_2)$alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure,

$R^2$ und $R^3$ gleiche oder verschiedene Reste Wasserstoff,
$(C_1$ bis $C_6)$-Alkyl, $(C_2$ bis $C_6)$-Alkenyl oder
$(C_6$ bis $C_{12})$-Aryl-$(C_1$ bis $C_4)$-alkyl bedeuten und

$R^4$ und $R^5$ die oben angegebene Bedeutung haben.

5. Verfahren gemäss einem der Ansprüche 1 bis 4, dadurch gekennzeichnet, dass Verbindungen der Formel I hergestellt werden, in welcher

n 1 oder 2 ist,

R Methyl, Ethyl, Cyclohexyl, tert.-Butoxycarbonylamino-$(C_1-C_4)$-alkyl oder Phenyl, das durch Phenyl, $(C_1$ bis $C_2)$-Alkyl, $(C_1$ oder $C_2)$-Alkoxy, Hydroxy, Fluor, Chlor, Brom, Amino, $(C_1$ bis $C_4)$-Alkylamino, Di-$(C_1$ bis $C_4)$alkylamino, Nitro und/oder Methylendioxy mono- oder disubstituiert oder im Falle von Methoxy, trisubstituiert sein kann,

$R^1$ Wasserstoff, $(C_1$ bis $C_3)$-Alkyl, $(C_2$ oder $C_3)$-Alkenyl, die gegebenanfalls geschützte Seitenkette von Lysin, Benzyl, 4-Methoxybenzyl, 4-Ethoxybenzyl, Phenethyl, 4-Amino-butyl oder Benzoylmethyl

$R^2$ und $R^3$ gleiche oder verschiedene Reste Wasserstoff,
$(C_1$ bis $C_4)$-Alkyl oder Benzyl bedeuten und

$R^4$ und $R^5$ die oben angegebene Bedeutung haben.

6. Verfahren zur Herstellung von Verbindungen der Formel III, in welcher

$R^1$ Wasserstoff oder $(C_1$ bis $C_6)$-Alkyl, das gegebenenfalls durch Amino, $(C_1$ bis $C_6)$-Acylamino oder Benzoylamino substituiert sein kann, $(C_2$ bis $C_6)$-Alkenyl, $(C_3$ bis $C_9)$-Cycloalkyl, $(C_5$ bis

$C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure,

$R^3$ Wasserstoff, ($C_1$ bis $C_6$)-Alkyl, ($C_2$ bis $C_6$)-Alkenyl oder ($C_6$ bis $C_{12}$-Aryl-($C_1$ bis $C_4$)-alkyl bedeuten und

$R^4$ und $R^5$ die bei Verbindungen der Formel I angegebene Bedeutung haben, dadurch gekennzeichnet, dass man α-Hydroxycarbonsäurederivate der Formel VII

```
          *a        *b
R3OOC-CH- N -C - CH-OH          (VII)
          |    |   |     |
         R4   R5 O  R1
```

in welcher $R^1$, $R^3$, $R^4$ und $R^5$ die obengenannten Bedeutungen haben, mit einem Trifluormethansulfonierungsmittel gegebenenfalls in Gegenwart einer Base umsetzt.

7. Verfahren gemäss Anspruch 6, dadurch gekennzeichnet, dass

$R^1$ Wasserstoff, ($C_1$ bis $C_3$)-Alkyl oder ($C_2$ bis $C_3$)-Alkenyl, die gegebenenfalls geschützte Seitenkette von Lysin, Benzyl, 4-Methoxybenzyl, 4-Ethoxybenzyl, Phenethyl, 4-Aminobutyl oder Benzoylmethyl

$R^3$ Wasserstoff, ($C_1$ bis $C_4$)-Alkyl oder Benzyl bedeuten und

$R^4$ und $R^5$ die bei Verbindungen der Formel I angegebene Bedeutung haben.

8. Verfahren gemäss Anspruch 6, dadurch gekennzeichnet, dass

$R^1$ Methyl, Ethyl, Phenyl, die gegebenenfalls acylierte Seitenkette von Lysin, Benzyl oder die O-($C_1$-$C_6$)-alkylierte Seitenkette von Tyrosin,

$R^3$ Wasserstoff, Methyl, Ethyl, tert. Butyl oder Benzyl bedeuten und

$R^4$ und $R^5$ die bei Verbindungen der Formel I angegebene Bedeutung haben.

**Claims for the Contracting States:**
BE, CH, DE, FR, GB, IT, LI, LU, NL, SE

1. A process for the preparation of compounds of the formula I

```
          *        *       *
R3OOC-CH-N - C -CH-NH-CH-(CH2)n-R          (I)
          |    |   ||  |          |
         R4  R5  O  R1        COOR2
```

in which

n is 1 or 2,

R denotes hydrogen, an optionally substituted aliphatic radical having 1 to 8 carbon atoms, an optionally substituted cycloaliphatic radical having 3-9 carbon atoms, an optionally substituted aromatic radical having 6-12 carbon atoms, an optionally substituted araliphatic radical having 7-14 carbon atoms, an optionally substituted cycloaliphatic-aliphatic radical having 7-14 carbon atoms, or a radical $OR^a$ or $SR^a$, in which

$R^a$ represents an optionally substituted aliphatic radical having 1-4 carbon atoms, an optionally substituted aromatic radical having 6-12 carbon atoms or an optionally substituted heteroaromatic radical having 5-12 ring atoms,

$R^1$ denotes hydrogen, an optionally substituted aliphatic radical having 1 to 6 carbon atoms, an optionally substituted cycloaliphatic radical having 3-9 carbon atoms, an optionally substituted cycloaliphatic-aliphatic radical having 4-13 carbon atoms, an optionally substituted aromatic radical having 6-12 carbon atoms, an optionally substituted araliphatic radical having 7-16 carbon atoms, an optionally substituted heteroaromatic radical having 5-12 ring atoms or the side-chain of an optionally protected naturally occurring α-aminoacid,

$R^2$ und $R^3$ are identifical or different and denote hydrogen, an optionally substituted aliphatic radical having 1-6 carbon atoms, an optionally substituted cycloaliphatic radical having 3-9 carbon atoms, an optionally substituted aromatic radical having 6-12 carbon atoms, an optionally substituted araliphatic radical having 7-16 carbon atoms, and

$R^4$ und $R^5$, together with the atoms carrying them, form a monocyclic, bicyclic or tricyclic heterocyclic ring system having 3 to 15 carbon atoms, which comprises reacting a compound of the formula II

```
                OSO2CF3
            *  /
R(CH2)n-CH                    (II)
               \
                COOR2
```

in which n, R and $R^2$ are as defined above with a compound of the formula IV

```
         *a        *b
R3OOC-CH-N - C -CH-NH2          (IV)
         |    |   ||  |
        R4  R5  O  R1
```

in which $R^1$, $R^3$, $R^4$ and $R^5$ are as defined above, or reacting a compound of the formula III

```
         *a        *b
R3OOC-CH-N - C -CH-OSO2CF3          (III)
         |    |   ||  |
        R4  R5  O  R1
```

in which $R^1$, $R^3$, $R^4$ and $R^5$ are as defined above, with a compound of the formula V

$$R\text{-}(CH_2)_n\text{-}\overset{*}{C}H(COOR^2)\text{-}NH_2 \qquad (V)$$

in which n, R and $R^2$ are as defined above, in the presence of at least one equivalent of a base which cannot react with compounds of the formulae II or III, splitting off, where appropriate, ester groups by hydrolysis or hydrogenolysis and, where appropriate, esterifying free carboxyl groups in a manner known per se.

2. The process as claimed in claim 1 in which is prepared a compound of the formula I in which $R^4$ and $R^5$, together with the atoms carrying them, represent an optionally substituted system from the series comprising pyrrolidine, piperidine, tetrahydroisoquinoline, decahydroisoquinoline, octahydroindole, octahydrocyclopenta-[b]pyrrole, 2-azabicyclo[2.2.2]octane, 2-azabicyclo-[2.2.1]-heptane, 2-azaspiro[4.5]decane, 2-azaspiro[4.4]-nonane, spiro[(bicyclo[2.2.1]heptane)-2,3-pyrrolidine], spiro[(bicyclo[2.2.2]octane)-2,3-pyrrolidine], 2-azatricyclo-[4.3.0.$1^{6,9}$]decane, decahydrocyclohepta[b]pyrrole, octahydroisoindole, octahydrocyclopenta[c]pyrrole, 2,3,3a,4,5,7a-hexahydroindole and tetrahydrothiazole.

3. The process as claimed in one of claims 1 or 2 in which is prepared a compound of the formula I in which

n is 1 or 2,

R denotes hydrogen, alkyl having 1-8 carbon atoms, alkenyl having 2-6 carbon atoms, cycloalkyl having 3-9 carbon atoms, aryl having 6-12 carbon atoms which can be monosubstituted, disubstituted or trisubstituted by $(C_1\text{-}C_4)$-alkyl, $(C_1\text{-}C_4)$-alkoxy, hydroxyl, halogen, nitro, amino, aminomethyl, $(C_1\text{-}C_4)$-alkylamino, di-$(C_1$--$C_4)$-alkylamino, $C_1\text{-}C_4)$-acylamino, preferably $(C_1\text{-}C_4)$-alkanoylamino, methylenedioxy, carboxyl, cyano and/or sulfamoyl, or alkoxy having 1-4 carbon atoms or aryloxy having 6-12 carbon atoms which can be substituted as described above for aryl, or monocyclic or bicyclic heteroaryloxy having 5-7 or 8-10 ring atoms respectively, 1 to 2 of these ring atoms being sulfur or oxygen atoms and/or 1 to 4 of these ring atoms being nitrogen, which can be substituted as described above for aryl, amino-$(C_1\text{-}C_4)$-alkyl, $(C_1\text{-}C_4)$-alkanoylamino-$(C_1\text{-}C_4)$-alkyl, $(C_7\text{-}C_{13})$--aroylamino-$(C_1\text{-}C_4)$-alkyl, $(C_1\text{-}C_4)$alkoxycarbonylamino-$(C_1\text{-}C_4)$-alkyl, $(C_6\text{-}C_{12})$-aryl-$(C_1\text{-}C_4)$--alkoxycarbonylamino-$(C_1\text{-}C_4)$-alkyl, $(C_6\text{-}C_{12})$--aryl$(C_1\text{-}C_4)$-alkylamino-$(C_1\text{-}C_4)$-alkyl, $(C_1\text{-}C_4)$-alkylamino-$(C_1\text{-}C_4)$-alkyl, di-$(C_1\text{-}C_4)$-alkylamino-$(C_1\text{-}C_4)$-alkyl, guanidino-$(C_1\text{-}C_4)$-alkyl, imidazolyl, indolyl, $(C_1\text{-}C_4)$-alkylthio, $(C_1\text{-}C_4)$--alkylthio-$(C_1\text{-}C_4)$-alkyl, $(C_6\text{-}C_{12})$-arylthio-$(C_1\text{-}C_4)$-alkyl, which can be substituted in the aryl moiety as described above for aryl, $(C_6\text{-}C_{12})$-aryl-$(C_1\text{-}C_4)$-alkylthio, which can be substituted in the aryl moiety as described above for aryl, carboxyl-$(C_1\text{-}C_4)$-alkyl, carboxyl, carbamoyl, carbamoyl-$(C_1\text{-}C_4)$-alkyl, $(C_1\text{-}C_4)$-alkoxycarbonyl-$(C_1\text{-}C_4)$-alkyl, $(C_6\text{-}C_{12}$ )-aryloxy-$(C_1\text{-}C_4)$-alkyl, which can be substituted in the aryl moiety as described above for aryl, or $(C_6\text{-}C_{12})$-aryl-$(C_1\text{-}C_4)$-alkoxy, which can be substituted in the aryl moiety as described above for aryl,

$R^1$ denotes hydrogen, alkyl having 1-6 carbon atoms, alkenyl having 2-6 carbon atoms, alkynyl havings 2-6 carbon atoms, cycloalkyl having 3-9 carbon atoms, cycloalkenyl having 5-9 carbon atoms, $(C_3\text{-}C_9)$-cycloalkyl-$(C_1\text{-}C_4)$-alkyl, $(C_5\text{-}C_9)$--cycloalkenyl-$C_1\text{-}C_4)$-alkyl, optionally partially hydrogenated aryl having 6-12 carbon atoms which can be substituted as described above for R, $(C_6\text{-}C_{12})$-aryl-$(C_1\text{-}C_4)$-alkyl or $(C_7\text{-}C_{13}$ )-aroyl--($C_1$ or $C_2$)-alkyl, both of which can be substituted as the previous aryl, monocyclic or bicyclic optionally partially hydrogenated, heteroaryl having 5-7 or 8-10 ring atoms respectively, 1 or 2 of these ring atoms being sulfur or oxygen atoms and/or 1 to 4 of these ring atoms being nitrogen atoms, which can be substituted as the previous aryl, or the side chain of an optionally protected naturally occurring α-aminoacid $R^1$-CH($NH_2$)-COOH,

$R^2$ and $R^3$ are identical or different and denote hydrogen, alkyl having 1-6 carbon atoms, alkenyl having 2-6 carbon atoms, di-$(C_1\text{-}C_4)$-alkylamino-$(C_1\text{-}C_4)$-alkyl, $(C_1\text{-}C_5)$-alkanoyloxy-$(C_1\text{-}C_4)$-alkyl, $(C_1\text{-}C_6)$-alkoxycarbonyloxy-$(C_1\text{-}C_4)$-alkyl, $(C_7\text{-}C_{13})$-aroyloxy-$(C_1\text{-}C_4)$-alkyl, $(C_6\text{-}C_{12})$-aryloxycarbonyloxy-$(C_1\text{-}C_4)$-alkyl, aryl having 6-12 carbon atoms $(C_6\text{-}C_{12})$-aryl-$(C_1\text{-}C_4)$-alkyl, $(C_3\text{-}C_9)$-cycloalkyl or $(C_3\text{-}C_9)$-cycloalkyl-$(C_1\text{-}C_4)$-alkyl, and

$R^4$ and $R^5$ have the meaning indicated above.

4. The process as claimed in one of claims 1 to 3 in which is prepared a compound of the formula I in which

n is 1 or 2

R denotes ($C_1$ to $C_6$)-alkyl, ($C_2$ to $C_6$)-alkenyl, ($C_3$ to $C_9$)-cycloalkyl, amino-$(C_1\text{-}C_4)$-alkyl, $(C_2\text{-}C_5)$-acylamino-$(C_1\text{-}C_4)$-alkyl, $(C_7\text{-}C_{13})$-aroylamino-$(C_1\text{-}C_4)$-alkyl, $(C_1\text{-}C_4)$-alkoxycarbonylamino-$(C_1\text{-}C_4)$-alkyl, ($C_6$ to $C_{12}$)-aryl-$(C_1\text{-}C_4)$-alkoxycarbonylamino-$(C_1\text{-}C_4)$-alkyl, ($C_6$ to $C_{12}$)-aryl which can be monosubstituted, disubstituted or trisubstituted by ($C_1$ to $C_4$)-alkyl, $(C_1\text{-}C_4)$-alkoxy, hydroxyl, halogen, nitro, amino, ($C_1$ to $C_4$)-alkylamino, di-($C_1$ to $C_4$)-alkylamino and/or methylenedioxy, or 3-indolyl,

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, each of which can be substituted by ($C_1$ to $C_4$)-alkyl, ($C_1$ or $C_2$)-alkoxy or halogen, $(C_6\text{-}C_{12})$-aryl-($C_1$ to $C_4$)-alkyl or $(C_7\text{-}C_{13})$-aroyl-$(C_1\text{-}C_2)$-alkyl, both of which can be substituted in the aroyl radical as previously defined, a monocyclic or bicyclic heterocyclic radical having 5 to 7 or 8 to 10 ring atoms, respectively, 1 or 2 of these ring atoms being sulfur or oxygen

atoms and/or 1 to 4 of these ring atoms being nitrogen atoms, or a side chain of a naturally occurring, optionally protected α-aminoacid,

$R^2$ and $R^3$ denote identical or different radicals, hydrogen, ($C_1$ to $C_6$)-alkyl, ($C_2$ to $C_6$)-alkenyl or ($C_6$ to $C_{12}$)-aryl-($C_1$ to $C_4$)-alkyl, and

$R^4$ and $R^5$ have the meanings indicated above.

5. The process as claimed in one of claims 1 to 4 in which is prepared a compound of the formula I in which

n is 1 or 2,

R denotes methyl, ethyl, cyclohexyl, tert.-butoxycarbonylamino-($C_1$-$C_4$)-alkyl or phenyl which can be monosubstituted or disubstituted or, in the case of methoxy, trisubstituted by phenyl, ($C_1$ to $C_2$)-alkyl, ($C_1$ or $C_2$)-alkoxy, hydroxyl, fluorine, chlorine, bromide, amino, ($C_1$ to $C_4$)-alkylamino, di-($C_1$ to $C_4$)-alkylamino, nitro and/or methylenedioxy,

$R^1$ denotes hydrogen, ($C_1$ to $C_3$)-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^2$ and $R^3$ denote identical or different radicals hydrogen, ($C_1$ to $C_4$)-alkyl or benzyl and

$R^4$ and $R^5$ have the meanings indicated above.

6. A compound of the formula III in which

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, each of which can be substituted by ($C_1$ to $C_4$)-alkyl, ($C_1$ or $C_2$)-alkoxy or halogen, ($C_6$-$C_{12}$)-aryl-($C_1$-$C_4$)-alkyl or ($C_7$-$C_{13}$)-aroyl-($C_1$-$C_2$)-alkyl, both of which can be substituted in the aryl radical as previously defined, a monocyclic or bicyclic heterocyclic radical having 5 to 7 or 8 to 10 ring aroms respectively, 1 or 2 of these ring atoms being sulfur or oxygen atoms and/or 1 to 4 of these ring atoms being nitrogen atoms, or a side chain of a naturally occurring, optionally protected α-aminoacid,

$R^3$ denotes hydrogen, ($C_1$ to $C_6$)-alkyl, ($C_2$ to $C_6$)-alkenyl or ($C_6$ .to $C_{12}$)-aryl-($C_1$ to $C_4$)-alkyl, and

$R^4$ and $R^5$ have the meanings indicated for the compound of the formula I.

7. A compound of the formula III as claimed in claim 6 in which

$R^1$ denotes hydrogen, ($C_1$ to $C_3$)-alkyl or ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^3$ denotes hydrogen, ($C_1$-$C_4$)-alkyl or benzyl, and

$R^4$ and $R^5$ have the meaning indicated for the compound of the formula I.

8. A compound of the formula III as claimed in claim 6 in which

$R^1$ denotes methyl, ethyl, phenyl, the optionally acylated side chain of lysine, benzyl or the O-($C_1$-$C_6$)-alkylated side chain of tyrosine,

$R^3$ denotes hydrogen, methyl, ethyl, tert.-butyl or benzyl and

$R^4$ and $R^5$ have the meaning indicated for the compounds of the formula I.

9. A process for the preparation of compounds of the formula III, in which $R^1$, $R^3$, $R^4$ and $R^5$ have the abovementioned meanings, which comprises reacting α-hydroxycarboxylic acid derivatives of the formula VII

```
         *a        *b
R3OOC-CH- N -C - CH-OH            (VII)
       |    |  |   |
       R4   R5 O   R1
```

in which $R^1$, $R^3$, $R^4$ and $R^5$ have the abovementioned meanings, with a trifluoromethanesulfonating agent, optionally in the presence of a base.

**Claims for the Contracting State:** AT

1. A process for the preparation of compounds of the formula I

```
          *       *     *
R3OOC-CH-N - C -CH-NH-CH-(CH2)n-R        (I)
       |   |  ||  |      |
       R4  R5 O   R1     COOR2
```

in which

n is 1 or 2,

R denotes hydrogen, an optionally substituted aliphatic radical having 1 to 8 carbon atoms, an optionally substituted cycloaliphatic radical having 3-9 carbon atoms, an optionally substituted aromatic radical having 6-12 carbon atoms, an optionally substituted araliphatic radical having 7-14 carbon atoms, an optionally substituted cycloaliphatic-aliphatic radical having 7-17 carbon atoms, or a radical $OR^a$ or $SR^a$, in which

$R^a$ represents an optionally substituted aliphatic radical having 1-4 carbon atoms, an optionally substituted aromatic radical having 6-12 carbon atoms or an optionally substituted heteroaromatic radical having 5-12 ring atoms,

$R^1$ denotes hydrogen, an optionally substituted aliphatic radical having 1 to 6 carbon atoms, an optionally substituted cycloaliphatic radical having 3-9 carbon atoms, an optionally substituted cycloaliphatic-aliphatic radical having 4-13 carbon atoms, an optionally substituted aromatic radical having 6-12 carbon atoms, an optionally substituted araliphatic radical having 7-16 carbon atoms, an optionally substituted heteroaromatic radical having 5-12 ring atoms or the side-chain of an optionally protected naturally occurring α-aminoacid,

$R^2$ and $R^3$ are identical or different and denote hydrogen, an optionally substituted aliphatic radical having 1-6 carbon atoms, an optionally substituted cycloaliphatic radical having 3-9 carbon atoms, an optionally substituted aromatic radical having 6-12 carbon atoms, an optionally substituted araliphatic radical having 7-16 carbon atoms, and

$R^4$ and $R^5$, together with the atoms carrying

them, form a monocyclic, bicyclic or tricyclic heterocyclic ring system having 3 to 15 carbon atoms, which comprises reacting a compound of the formula II

```
              OSO2CF3
           * /
R(CH2)n-CH                          (II)
             \
              COOR2
```

in which n, R and $R^2$ are as defined above with a compound of the formula IV

```
       *a       *b
R3OOC-CH-N - C -CH-NH2              (IV)
       |    |  ||  |
       R4  R5  O  R1
```

in which $R^1$, $R^3$, $R^4$ and $R^5$ are as defined above, or reacting a compound of the formula III

```
       *a       *b
R3OOC-CH-N - C -CH-OSO2CF3          (III)
       |    |  ||  |
       R4  R5  O  R1
```

in which $R^1$, $R^3$, $R^4$ and $R^5$ are as defined above, with a compound of the formula V

```
          *
R-(CH2)n-CH-NH2                     (V)
          |
          COOR2
```

in which n, R and $R^2$ are as defined above, in the presence of at least one equivalent of a base which cannot react with compounds of the formulae II or III, splitting off, where appropriate, ester groups by hydrolysis or hydrogenolysis and, where appropriate, esterifying free carboxyl groups in a manner known per se.

2. The process as claimed in claim 1 in which is prepared a compound of the formula I in which $R^4$ and $R^5$, together with the atoms carrying them, represent an optionally substituted system from the series comprising pyrrolidine, piperidine, tetrahydrosioquinoline, decahydroisoquinoline, octahydroindole, octahydrocyclopenta-[b]pyrrole, 2-aza-bicyclo[2.2.2]octane, 2-azabicyclo-[2.2.1]-heptane, 2-azaspiro[4.5]decane, 2-azaspiro[4.4]-nonane, spiro-[(bicyclo[2.2.1]heptane)-2,3-pyrrolidine], spiro](bicyclo[2.2.2]octane)-2,3-pyrrolidine], 2-azatricyclo-[4.3.0.1$^{6,9}$]decane, decahydrocyclohepta[b]pyrrole, octahydroisoindole, octahydrocyclopenta[c]pyrrole, 2,3,3a,4,5,7a-hexahydroindole and tetrahydrothiazole.

3. The process as claimed in one of claims 1 or 2 in which is prepared a compound of the formula I in which

n is1 or 2,

R denotes hydrogen, alkyl having 1-8 carbon atoms, alkenyl having 2-6 carbon atoms, cycloalkyl having 3-9 carbon atoms, aryl having 6-12 carbon atoms which can be monosubstituted, disubstituted or trisubstituted by ($C_1$-$C_4$)-alkyl, ($C_1$-$C_4$)-alkoxy, hydroxyl, halogen, nitro, amino, aminomethyl, ($C_1$-$C_4$)-alkylamino, di-($C_1$-$C_4$)-alkylamino, ($C_1$-$C_4$)-acylamino, preferably ($C_1$-$C_4$)-alkanoylamino, methylenedioxy, carboxyl, cyano and/or sulfamoyl, or alkoxy having 1-4 carbon atoms or aryloxy having 6-12 carbon atoms which can be substituted as described above for aryl, or monocyclic or bicyclic heteroaryloxy having 5-7 or 8-10 ring atoms respectively, 1 to 2 of these ring atoms being sulfur or oxygen atoms and/or 1 to 4 of these ring atoms being nitrogen, which can be substituted as described above for aryl, amino-($C_1$-$C_4$)-alkyl, ($C_1$-$C_4$)-alkanoylamino-($C_1$-$C_4$-alkyl, ($C_7$-$C_{13}$)-aroylamino-($C_1$-$C_4$)-alkyl, $C_1$-$C_4$)-alkoxycarbonylamino-($C_1$-$C_4$)-alkyl, ($C_6$-$C_{12}$)-aryl-($C_1$-$C_4$)-alkoxycarbonylamino-($C_1$-$C_4$)-alkyl, ($C_6$-$C_{12}$)-aryl($C_1$-$C_4$)-alkylamino-($C_1$-$C_4$)-alkyl, ($C_1$-$C_4$)-alkylamino-($C_1$-$C_4$)-alkyl, di-($C_1$-$C_4$)-alkylamino-($C_1$-$C_4$)-alkyl, guanidino-($C_1$-$C_4$)-alkyl, imidazolyl, indolyl, ($C_1$-$C_4$)-alkylthio, ($C_1$-$C_4$)-alkylthio-($C_1$-$C_4$)-alkyl, ($C_6$-$C_{12}$)-arylthio($C_1$-$C_4$)-alkyl, which can be substituted in the aryl moiety as described above for aryl, ($C_6$-$C_{12}$)-aryl-($C_1$-$C_4$)-alkylthio, which can be substituted in the aryl moiety as described above for aryl, carboxyl-($C_1$-$C_4$)-alkyl, carboxyl, carbamoyl, carbamoyl-($C_1$-$C_4$)-alkyl, ($C_1$-$C_4$)-alkoxycarbonyl-($C_1$-$C_4$)-alkyl, ($C_6$-$C_{12}$)-aryloxy-($C_1$-$C_4$) alkyl, which can be substituted in the aryl moiety as described above for aryl, or ($C_6$-$C_{12}$)-aryl-($C_1$-$C_4$)-alkoxy, which can be substituted in the aryl moiety as described above for aryl),

$R^1$ denotes hydrogen, alkyl having 1-6 carbon atoms, alkenyl having 2-6 carbon atoms, alkynyl having 2-6 carbon atoms, cycloalkyl having 3-9 carbon atoms, cycloalkenyl having 5-9 carbon atoms, ($C_3$-$C_9$)-cycloalkyl-($C_1$-$C_4$)-alkyl, ($C_5$-$C_9$)-cycloalkenyl-($C_1$-$C_4$)-alkyl, optionally partially hydrogenated aryl having 6-12 carbon atoms which can be substituted as described above for R, ($C_6$-$C_{12}$)-aryl-($C_1$-$C_4$)-alkyl or ($C_7$-$C_{13}$)-aroyl-($C_1$ or $C_2$)-alkyl, both of which can be substituted as the previous aryl, monocyclic or bicyclic optionally partially hydrogenated, heteroaryl having 5-7 or 8-10 ring atoms respectively, 1 or 2 of these ring atoms being sulfur or oxygen atoms and/or 1 to 4 of these ring atoms being nitrogen atoms, which can be substituted as the previous aryl, or the side chain of an optionally protected naturally occurring α-aminoacid $R^1$-$CH(NH_2)$-COOH,

$R^2$ and $R^3$ are identical or different and denote hydrogen, alkyl having 1-6 carbon atoms, alkenyl having 2-6 carbon atoms, di-($C_1$-$C_4$)-alkylamino-($C_1$-$C_4$)-alkyl, ($C_1$-$C_5$)-alkanoyloxy-($C_1$-$C_4$)-alkyl, ($C_1$-$C_6$)-alkoxycarbonyloxy-($C_1$-$C_4$)-alkyl, ($C_7$-$C_{13}$)-aroyloxy-($C_1$-$C_4$)-alkyl, ($C_6$-$C_{12}$)-aryloxycarbonyloxy-($C_1$-$C_4$)-alkyl, aryl having 6-12 carbon atoms, ($C_6$-$C_{12}$)-aryl-($C_1$-$C_4$)-alkyl, ($C_3$-$C_9$)-cycloalkyl or ($C_3$-$C_9$)-cycloalkyl-($C_1$-$C_4$)-alkyl, and

$R^4$ and $R^5$ have the meanings indicated above.

4. The process as claimed in one of claims 1 to 3 in which is prepared a compound of the formula I in which

n is 1 or 2,

R denotes ($C_1$ to $C_6$)-alkyl, ($C_2$ to $C_6$)-alkenyl, ($C_3$ to $C_9$)-cycloalkyl, amino-($C_1$-$C_4$)-alkyl, ($C_2$-$C_5$)-acylamino-($C_1$-$C_4$)-alkyl, ($C_7$-$C_{13}$)-aroylamino-($C_1$-$C_4$)-alkyl, ($C_1$-$C_4$)-alkoxycarbonylamino-($C_1$-$C_4$)-alkyl, ($C_6$ to $C_{12}$)-aryl-($C_1$-$C_4$)-alkoxycarbonylamino-($C_1$-$C_4$)-alkyl, ($C_6$ to $C_{12}$)-aryl which can be monosubstituted, disubstituted or trisubstituted by ($C_1$ to $C_4$)-alkyl, ($C_1$-$C_4$)-alkoxy, hydroxyl, halogen, nitro, amino, ($C_1$ to $C_4$)-alkylamino, di-($C_1$ to $C_4$)-alkylamino and/or methylenedioxy, or 3-Indolyl,

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, each of which can be substituted by ($C_1$ to $C_4$)-alkyl, ($C_1$ or $C_2$)-alkoxy or halogen, ($C_6$-$C_{12}$)-aryl-($C_1$ to $C_4$)-alkyl or ($C_7$-$C_{13}$)-aroyl-($C_1$-$C_2$)-alkyl, both of which can be substituted in the aroyl radical as previously defined, a monocyclic or bicyclic heterocyclic radical having 5 to 7 or 8 to 10 ring atoms, respectively, 1 or 2 of these ring atoms being sulfur or oxygen atoms and/or 1 to 4 of these ring atoms being nitrogen atoms, or a side chain of a naturally occurring, optionally protected α-aminoacid,

$R^2$ and $R^3$ denote identical or different radicals, hydrogen, ($C_1$ to $C_6$)-alkyl, ($C_2$ to $C_6$)-alkenyl or ($C_6$ to $C_{12}$)-aryl-($C_1$ to $C_4$)-alkyl, and

$R^4$ and $R^5$ have the meanings indicated above.

5. The process as claimed in one of claims 1 to 4 in which is prepared a compound of the formula I in which

n is 1 or 2,

R denotes methyl, ethyl, cyclohexyl, tert.-butoxycarbonylamino-($C_1$-$C_4$)-alkyl or phenyl which can be monosubstituted or disubstituted or, in the case of methoxy, trisubstituted by phenyl, ($C_1$ to $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy,

$R^1$ denotes hydrogen, ($C_1$ to $C_3$)-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^2$ and $R^3$ denote identical or different radicals hydrogen, ($C_1$ to $C_4$)-alkyl or benzyl and

$R^4$ and $R^5$ have the meanings indicated above.

6. A process for the preparation of compounds of the formula III

in which

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, each of which can be substituted by ($C_1$ to $C_4$)-alkyl, ($C_1$ or $C_2$)-alkoxy or halogen, ($C_6$-$C_{12}$)-aryl-($C_1$-$C_4$)-alkyl or ($C_7$-$C_{13}$)-aroyl-($C_1$-$C_2$)-alkyl, both of which can be substituted in the aryl radical as previously defined, a monocyclic or bicyclic heterocyclic radical having 5 to 7 or 8 to 10 ring aroms respectively, 1 or 2 of these ring atoms being sulfur or oxygen atoms and/or 1 to 4 of these ring atoms being nitrogen atoms, or a side chain of a naturally occurring, optionally protected α-aminoacid,

$R^3$ denotes hydrogen, ($C_1$ to $C_6$)-alkyl, ($C_2$ to $C_6$)-alkenyl or ($C_6$ to $C_{12}$)-aryl-($C_1$ to $C_4$)-alkyl, and

$R^4$ and $R^5$ have the meanings indicated for the compound of the formula I which comprises reacting α-hydroxycarboxylic acid derivatives of the formula VII

```
        *a        *b
R3OOC-CH- N -C - CH-OH          (VII)
        |    |  ||  |
        R4   R5 O   R1
```

in which $R^1$, $R^3$, $R^4$ and $R^5$ have the abovementioned meanings, with a trifluoromethanesulfonating agent, optionally in the presence of a base.

7. A process as claimed in claim 6 in which

$R^1$ denotes hydrogen, ($C_1$ to $C_3$)-alkyl or ($C_2$ or $C_3$)-alkenyl, the optionally protected side chain of lysine, benzyl, 4-methoxybenzyl, 4-ethoxybenzyl, phenethyl, 4-aminobutyl or benzoylmethyl,

$R^3$ denotes hydrogen, ($C_1$-$C_4$)-alkyl or benzyl, and

$R^4$ and $R^5$ have the meaning indicated for the compound of the formula I.

8. A process as claimed in claim 6 in which

$R^1$ denotes methyl, ethyl, phenyl, the optionally acylated side chain of lysine, benzyl or the O-($C_1$-$C_6$)-alkylated side chain of tyrosine,

$R^3$ denotes hydrogen, methyl, ethyl, tert.-butyl or benzyl and

$R^4$ and $R^5$ have the meaning indicated for the compounds of the formula I.

**Revendications pour les Etats contractants:** BE, CH, DE, FR, GB, IT, LI, LU, NL, SE

1. Procédé de préparation de composés de formule I

```
        *       *      *
R3OOC-CH-N - C -CH-NH-CH-(CH2)n-R        (I)
       |   |  ||  |      |
       R4  R5 O   R1     COOR2
```

dans laquelle

n est 1 ou 2

R représente l'hydrogène, un groupe aliphatique éventuellement substitué ayant de 1 à 8 atomes de carbone, un groupe cycloaliphatique éventuellement substitué ayant de 3 à 9 atomes de carbone,

un groupe aromatique éventuellement substitué ayant de 6 à 12 atomes de carbone,
un groupe araliphatique éventuellement substitué ayant de 7 à 14 atomes de carbone,
un groupe cycloaliphatique-aliphatique éventuellement substitué ayant de 7 à 14 atomes de carbone,
un groupe $OR^a$ ou $SR^a$ dans lequel

$R^a$ représente un groupe aliphatique éventuellement substitué ayant de 1 à 4 atomes de carbone, un groupe aromatique éventuellement substitué ayant de 6 à 12 atomes de carbone ou un groupe hétéroaromatique éventuellement substitué ayant de 5 à 12 atomes de carbone dans le noyau,

$R^1$ représente l'hydrogène,
un groupe aliphatique éventuellement substitué, ayant de 1 à 6 atomes de carbone,
un groupe cycloaliphatique éventuellement substitué, ayant de 3 à 9 atomes de carbone,
un groupe cycloaliphatique-aliphatique éventuellement substitué ayant de 4 à 13 atomes de carbone,
un groupe aromatique éventuellement substitué ayant de 6 à 12 atomes de carbone,
un groupe araliphatique éventuellement substitué ayant de 7 à 16 atomes de carbone,
un groupe hétéroaromatique éventuellement substitué ayant de 5 à 12 atomes dans le noyau ou
la chaîne latérale d'un α-amino acide naturel, éventuellement substitué,

$R^2$ et $R^3$ sont identiques ou diffétents et représentent l'hydrogène,
un groupe aliphatique éventuellement substitué ayant de 1 à 6 atomes de carbone,
un groupe cycloaliphatique éventuellement substitué ayant de 3 à 9 atomes de carbone,
un groupe aromatique éventuellement substitué ayant de 6 à 12 atomes de carbone,
un groupe araliphatique éventuellement substitué ayant de 7 à 16 atomes de carbone, et

$R^4$ et $R^5$ forment ensemble avec les atomes les portant un système hétérocyclique, mono-, bi- ou tricyclique ayant de 3 à 15 atomes de carbone,

ce procédé étant caractérisé en ce qu'on fair réagir un composé de formule II

```
             OSO2CF3
           * /
R(CH2)n-CH                    (II)
             \
             COOR2
```

dans laquelle n, R et $R^2$ ont les significations indiquées ci-dessus, avec un composé de formule IV

```
       *a          *b
R3OOC-CH-N - C -CH-NH2        (IV)
       |   |   ||  |
       R4  R5  O   R1
```

dans laquelle $R^1$, $R^3$, $R^4$ et $R^5$ ont les significations ci-dessus, ou on fait réagir un composé répondant à la formule III

```
       *a          *b
R3OOC-CH-N - C -CH-OSO2CF3    (III)
       |   |   ||  |
       R4  R5  O   R1
```

dans laquelle $R^1$, $R^3$, $R^4$ et $R^5$ ont les significations indiquées ci-dessus avec un composé répondant à la formule V

```
           *
R-(CH2)n-CH-NH2               (V)
           |
           COOR2
```

dans laquelle n, R et $R^2$ ont les significations indiquées ci-dessus, en présence d'au moins un équivalent d'une base qui ne réagit pas avec des composés répondant aux formules II et III, on élimine éventuellement les groupes ester par hydrolyse ou hydrogénolyse et on estérifie éventuellement les groupes carboxy libres d'une manière connue en soi.

2. Procédé selon la revendication 1, caractérisé en ce qu'on prépare des composés de formule I, dans lesquels $R^4$ et $R^5$ forment ensemble avec les atomes de carbone les portant un système éventuellement substitué de la série des: pyrrolidine, pipéridine, tétrahydroisoquinoléine, décahydroisoquinoléine, octahydroindole, octahydrocyclopenta[b]pyrrole, 2-aza-bicyclo[2.2.2]octane, 2-azabicyclo[2.2.1]-heptane, 2-azaspiro[4.5)décane, 2-azaspiro[4.4]-nonane, spiro[(bicyclo[2.2.1]heptane)-2,3-pyrrolidine], spiro [(bicyclo[2.2.2]octane)-2,3-pyrrolidine], 2-azatricyclo[4,3,0,$1^{6,9}$]décane, décahydrocyclohepta[b]pyrrole, octahydroisoindole, octahydrocyclopenta[c]pyrrole, 2,3,3a,4,5,7a-hexahydroindole et tétrahydrothiazole.

3. Procédé selon la revendication 1 ou 2, caractérisé en ce qu'on prépare des composés de formule I, dans lesquels:

n est 1 ou 2,

R représente l'hydrogène,
un groupe alcoyle ayant de 1 à 8 atomes de carbone,
un groupe alcényle ayant de 2 à 6 atomes de carbone,
un groupe cycloalcoyle ayant de 3 à 9 atomes de carbone,
un groupe aryle ayant de 6 à 12 atomes de carbone qui ne peut être mono-, di- ou trisubstitué par un groupe alcoyle en $C_1$-$C_4$, alcoxy en $C_1$-$C_4$, hydroxy, halogéno, nitro, amino, aminométhyle, alcoyl($C_1$-$C_4$)-amino, di-alcoyl($C_1$-$C_4$)-amino, acyl($C_1$-$C_4$)-amino, de préférence alcanoyl ($C_1$-$C_4$)-amino, méthylènedioxy, carboxy, cyano et/ou sulfamoyle,
un groupe alcoxy ayant de 1 à 4 atomes de carbone,
un groupe aryloxy ayant de 6 à 12 atomes de carbone, qui peut être substitué comme décrit pour le groupe aryle,
un groupe hétéroaryloxy mono- ou bicyclique, ayant de 5 à 7 ou de 8 à 10 atomes dans le noyau, dont de 1 à 2 atomes du noyau représentent des

atomes de soufre ou d'oxygène et/ou de 1 a 4 atomes du noyau représentent des atomes d'azote qui peut être substitué comme décrit ci-déssus pour le groupe aryle,
un groupe amino-alcoyle en $C_1$-$C_4$,
un groupe alcanoyl($C_1$-$C_4$)-amino-alcoyle ($C_1$-$C_4$),
un groupe aroyl($C_7$-$C_{13}$)-amino-alcoyle ($C_1$-$C_4$),
un groupe alcoxy($C_1$-$C_4$)-carbonylamino-alcoyle ($C_1$-$C_4$),
un groupe aryl($C_6$-$C_{12}$)-alcoxy($C_1$-$C_4$)-carbonyl-amino-alcoyle ($C_1$-$C_4$),
un groupe aryl($C_6$-$C_{12}$)-alcoylamino($C_1$-$C_4$)-alcoyle ($C_1$-$C_4$),
un groupe alcoyl($C_1$-$C_4$)-amino-alcoyle ($C_1$-$C_4$),
un groupe di-alcoyl($C_1$-$C_4$)-amino-alcoyle ($C_1$-$C_4$),
un groupe guanidino-alcoyle ($C_1$-$C_4$),
un groupe imidazolyle, indolyle,
un groupe alcoyl($C_1$-$C_4$)-thio,
un groupe alcoyl($C_1$-$C_4$)-thio-alcoyle ($C_1$-$C_4$),
un groupe aryl($C_6$-$C_{12}$)-thio-alcoyle ($C_1$-$C_4$), qui peut être substitué dans la partie aryle comme décrit ci-dessus pour le groupe aryle,
un groupe aryl($C_6$-$C_{12}$)-alcoyle($C_1$-$C_4$)-thio, qui peut être substitué dans la partie aryle, comme décrit ci-dessus pour le groupe aryle,
un groupe carboxy-alcoyle ($C_1$-$C_4$),
un groupe carboxy, carbamoyle,
un groupe carbamoyl-alcoyle ($C_1$-$C_4$),
un groupe alcoxy($C_1$-$C_4$)-carbonyl-alcoyle ($C_1$-$C_4$),
un groupe aryloxy($C_6$-$C_{12}$ )-alcoyle ($C_1$-$C_4$), qui-peut être substitué dans la partie aryle comme décrit ci-dessus pour le groupe aryle, ou
un groupe aryl($C_6$-$C_{12}$)-alcoxy ($C_1$-$C_4$), qui peut être substitué dans la partie aryle comme décrit ci-dessus pour le groupe aryle,

$R^1$ représente l'hydrogène,
un groupe alcoyle ayant de 1 à 6 atomes de carbone,
un groupe alcényle ayant de 2 à 6 atomes de carbone,
un groupe alcynyle ayant de 2 à 6 atomes de carbone,
un groupe cycloalcoyle ayant de 3 à 9 atomes de carbone,
un groupe cycloalcényle ayant de 5 à 9 atomes de carbone,
un groupe cycloalcoyl($C_3$-$C_9$)-alcoyle ($C_1$-$C_4$),
un groupe cycloalcényl($C_5$-$C_9$)-alcoyle ($C_1$-$C_4$),
un groupe aryle éventuellement partiellement hydrogéné, ayant de 6 à 12 atomes de carbone, qui peut être substitué comme décrit pour R ci-dessus,
un groupe aryl($C_6$-$C_{12}$)-alcoyle ($C_1$-$C_4$) ou aroyl($C_7$-$C_{13}$)-alcoyle ($C_1$ ou $C_2$) qui peuvent tous les deux être substitués comme le groupe aryle précédent,
un groupe hétéroaryle mono- ou bicyclique, éventuellement partiellement hydrogéné, ayant de 5 à 7 ou de 8 à 10 atomes dans le noyau, dont de 1 à 2 atomes du noyau représentent des atomes de soufre ou d'oxygène et/ou de 1 à 4 atomes du noyau représentent des atomes d'azote, qui peut être substitué comme le groupe aryle précédent, ou
la chaîne latérale d'un α-amino acide $R^1$-CH($HN_2$)-COOH d'origine naturelle, éventuellement protégé,

$R^2$ et $R^3$ sont identiques ou différents et reprepréséntent l'hydrogène,
un groupe alcoyle ayant de 1 à 6 atomes de carbone,
un groupe alcényle ayant de 2 à 6 atomes de carbone,
un groupe di-alcoyl($C_1$-$C_4$)-amino-alcoyle ($C_1$-$C_4$),
un groupe alcanoyloxy($C_1$-$C_5$)-alcoyle ($C_1$-$C_4$),
un groupe alcoxy($C_1$-$C_6$)-carbonyloxy-alcoyle ($C_1$-$C_4$),
un groupe aroyloxy($C_7$-$C_{13}$)-alcoyle ($C_1$-$C_4$),
un groupe aryloxy($C_6$-$C_{12}$)-carbonyloxy-alcoyle ($C_1$-$C_4$),
un groupe aryle ayant de 6 à 12 atomes de carbone,
un groupe aryl($C_6$-$C_{12}$)-alcoyle ($C_1$-$C_4$),
un groupe cycloalcoyle en $C_3$-$C_9$ ou
un groupe cycloalcoyl($C_3$-$C_9$)-alcoyle ($C_1$-$C_4$), et

$R^4$ et $R^5$ ont la signification indiquée ci-dessus.

4. Procédé selon l'une quelconque des revendications 1 à 3, caractérisé en ce qu'on prépare des composés de formula I, dans lesquels:

n est 1 ou 2,

R représente un groupe alcoyle en $C_1$-$C_6$, alcényle en $C_2$-$C_6$, cycloalcoyle en $C_3$-$C_9$, aminoalcoyle en $C_1$-$C_4$, acyl($C_2$-$C_5$)-aminoalcoyle ($C_1$-$C_4$), aroyl($C_7$-$C_{13}$)-aminoalcoyle ($C_1$-$C_4$), alcoxy ($C_1$-$C_4$)-carbonylamino-alcoyle ($C_1$-$C_4$), aryl-($C_6$-$C_{12}$)-alcoxy($C_1$-$C_4$)-carbonylamino-alcoyle ($C_1$-$C_4$), aryle en $C_6$-$C_{12}$ qui peut être mono-, di- ou trisubstitué par un groupe alcoyle en $C_1$-$C_4$, alcoxy en $C_1$-$C_4$, hydroxy, halogéno, nitro, amino, alcoyl($C_1$-$C_4$)-amino, di-alcoyl($C_1$-$C_4$)-amino et/ou méthylènedioxy,
ou un groupe 3-indolyle,

$R^1$ représente l'hydrogène ou un groupe alcoyle en $C_1$-$C_6$ qui peut eventuellement être substitué par un groupe amino, acyl($C_1$-$C_6$)-amino ou benzoylamino; un groupe alcényle en $C_2$-$C_6$, cycloalcoyle en $C_3$-$C_9$, cycloalcényle en $C_5$-$C_9$, cycloalcoyl($C_3$-$C_7$)-alcoyle ($C_1$-$C_4$), aryle en $C_6$-$C_{12}$, ou aryle partiellement hydrogéné, qui peuvent chacun être substitués par un groupe alcoyle en $C_1$-$C_4$, alcoxy en $C_1$ ou $C_2$ ou par un halogène; un groupe aryl($C_6$-$C_{12}$)-alcoyle ($C_1$-$C_4$) ou aroyl ($C_7$-$C_{13}$)-alcoyle ($C_1$-$C_2$), qui peuvent être tous les deux substitués comme défini ci-dessus pour le groupe aryle; un groupe hétérocyclique mono- ou bicyclique ayant de 5 à 7 ou de 8 à 10 atomes dans le noyau, dont de 1 à 2 atomes du noyau sont des atomes de soufre ou d'oxygène et/ou de 1 à 4 atomes du noyau sont des atomes d'azote; ou une chaîne latérale d'un α-amino acide d'origine naturelle, éventuellement protégé,

$R^2$ et $R^3$ sont identiques ou différents et représentent l'hydrogène, un groupe alcoyle en $C_1$-$C_6$, alcényle en $C_2$-$C_6$ ou aryl($C_6$-$C_{12}$)-alcoyle ($C_1$-$C_4$) et

$R^4$ et $R^5$ ont la signification indiquée ci-dessus.

5. Procédé selon l'une quelconque des revendications 1 à 4, caractérisé en ce qu'on prépare des composés de formule I, dans lesquels:

n est 1 ou 2,

R représente un groupe méthyle, éthyle, cyclohexyle, tert-butoxy-carbonylamino-alcoyle ($C_1$-$C_4$) ou phényle, qui peut être mono- ou disubstitué par un groupe phényle, alcoyle en $C_1$ à $C_2$, alcoxy en $C_1$ ou $C_2$, hydroxy, fluoro, bromo, chloro, amino, alcoylamino en $C_1$-$C_4$, di-alcoyl($C_1$-$C_4$)-amino, nitro et/ou méthylènedioxy, ou peut être trisubstitué dans le cas d'un groupe méthoxy,

$R^1$ représente l'hydrogène, un groupe alcoyle en $C_1$-$C_3$, alcényle en $C_2$ ou $C_3$, la chaîne latérale éventuellement protégée de la lysine, un groupe benzyle, 4-méthoxybenzyle, 4-éthoxybenzyle, phénéthyle, 4-aminobutyle ou benzoylméthyle,

$R^2$ et $R^3$ sont identiques ou différents et représentent l'hydrogène, un groupe alcoyle en $C_1$-$C_4$ ou benzyle, et

$R^4$ et $R^5$ ont la signification indiquée ci-dessus.

6. Composé de formule III, dans lequel:

$R^1$ représente l'hydrogène ou un groupe alcoyle en $C_1$-$C_6$, qui peut être éventuellement substitué par un groupe amino, acyl-($C_1$-$C_6$)amino ou benzoylamino; un groupe alcényle en $C_2$-$C_6$, cycloalcoyle en $C_3$-$C_9$, cycloalcényle en $C_5$-$C_9$, cycloalcoyl-($C_3$-$C_7$)-alcoyle ($C_1$-$C_4$), aryle en $C_6$-$C_{12}$ ou aryle partiellement hydrogéné, qui peuvent chacun être substitués par un groupe alcoyle en $C_1$-$C_4$, alcoxy en $C_1$ ou $C_2$ ou par un halogène; un groupe aryl($C_6$-$C_{12}$)-alcoyle ($C_1$-$C_4$) ou aroyl($C_7$-$C_{13}$)-alcoyle ($C_1$-$C_2$) qui peuvent être tous les deux substitués dans la partie aryle comme défini précédemment; un groupe hétérocyclique mono- ou bicyclique ayant de 5 à 7 ou de 8 à 10 atomes dans le noyau, dont de 1 à 2 atomes du noyau sont des atomes de soufre ou d'oxygène et/ou de 1 à 4 atomes du noyau sont des atomes d'azote, ou une chaîne latérale d'un α-amino acide d'origine naturelle, éventuellement protégé,

$R^3$ représente l'hydrogène, un groupe alcoyle en $C_1$-$C_6$, alcényle en $C_2$-$C_6$ ou aryl($C_6$-$C_{12}$)-alcoyle ($C_1$-$C_4$) et

$R^4$ et $R^5$ ont la signification indiquée pour les composés de formule I.

7. Composé de formule III selon la revendication 6, dans lequel:

$R^1$ représente l'hydrogène, un groupe alcoyle en $C_1$-$C_3$ ou alcényle en $C_2$ à $C_3$, la chaîne latérale éventuellement protégée de la lysine, un groupe benzyle, 4-méthoxybenzyle, 4-éthoxybenzyle, phénéthyle, 4-aminobutyle ou benzoylméthyle,

$R^3$ représente l'hydrogène, un groupe alcoyle en $C_1$-$C_4$ ou benzyle, et

$R^4$ et $R^5$ ont la signification indiquée pour les composés de formule I.

8. Composé de formule III selon la revendication 6, dans lequel:

$R^1$ représente un groupe méthyle, éthyle, phényle, la chaîne latérale éventuellement acylée de la lysine, un groupe benzyle ou la chaîne latérale O-alcoylée en $C_1$-$C_6$ de la tyrosyne,

$R^3$ représente l'hydrogène, un groupe méthyle, éthyle, tert-butyle ou benzyle et

$R^4$ et $R^5$ ont la signification indiquée pour les composés de formule I.

9. Procédé de préparation de composés de formule III, dans lesquels $R^1$, $R^3$, $R^4$ et $R^5$ ont les significations indiquées ci-dessus, caractérisé en ce qu on fait réagir des dérivés d'acides α-hydroxy-carboxyliques de formule VII

```
        *a        *b
R³OOC-CH- N -C - CH-OH          (VII)
      |   |  |   |
      R⁴  R⁵ O   R¹
```

dans laquelle $R^1$, $R^3$, $R^4$ et $R^5$ ont les significations indiquées ci-dessus, avec un agent de trifluorométhanesulfonation, éventuellement en présence d'une base.

**Revendications pour l'Etat contractant:** AT

1. Procédé de préparation de composés de formule I

```
      *       *      *
R³OOC-CH-N - C -CH-NH-CH-(CH₂)n-R        (I)
      |   |  ||  |     |
      R⁴  R⁵ O   R¹    COOR²
```

dans laquelle

n est 1 ou 2

R représente l'hydrogène,
un groupe aliphatique éventuellement substitué ayant de 1 à 8 atomes de carbone,
un groupe cycloaliphatique éventuellement substitué ayant de 3 à 9 atomes de carbone,
un groupe aromatique éventuellement substitué ayant de 6 à 12 atomes de carbone,
un groupe araliphatique éventuellement substitué ayant de 7 à 14 atomes de carbone,
un groupe cycloaliphatique-aliphatique éventuellement substitué ayant de 7 à 14 atomes de carbone,
un groupe $OR^a$ ou $SR^a$ dans lequel

$R^a$ représente un groupe aliphatique éventuellement substitué ayant de 1 à 4 atomes de carbone, un groupe aromatique éventuellement substitué ayant de 6 à 12 atomes de carbone ou un groupe hétéroaromatique éventuellement substitué ayant de 5 à 12 atomes de carbone dans le noyau,

$R^1$ représente l'hydrogène,
un groupe aliphatique éventuellement substitué, ayant de 1 à 6 atomes de carbone,
un groupe cycloaliphatique éventuellement substitué, ayant de 3 à 9 atomes de carbone,
un groupe cycloaliphatique-aliphatique éventuellement substitué ayant de 4 à 13 atomes de carbone,
un groupe aromatique éventuellement substitué ayant de 6 à 12 atomes de carbone,

un groupe araliphatique éventuellement substitué ayant de 7 à 16 atomes de carbone,
un groupe hétéroaromatique éventuellement substitué ayant de 5 à 12 atomes dans le noyau ou
la chaîne latérale d'un α-amino acide naturel, éventuellement substitué,
$R^2$ et $R^3$ sont identiques ou différents et représentent l'hydrogène, un groupe aliphatique éventuellement substitué ayant de 1 à 6 atomes de carbone,
un groupe cycloaliphatique éventuellement substitué ayant de 3 à 9 atomes de carbone,
un groupe aromatique éventuellement substitué ayant de 6 à 12 atomes de carbone,
un groupe araliphatique éventuellement substitué ayant de 7 à 16 atomes de carbone, et
$R^4$ et $R^5$ forment ensemble avec les atomes les portant un système hétérocyclique, mono-, bi- ou tricyclique ayant de 3 à 15 atomes de carbone,

ce procédé étant caractérisé en ce qu'on fait réagir un composé de formule II

```
             OSO2CF3
         *  /
R(CH2)n-CH                    (II)
            \
             COOR2
```

dans laquelle n, R et $R^2$ ont les significations indiquées ci-dessus, avec un composé de formule IV

```
       *a         *b
R3OOC-CH-N - C -CH-NH2                  (IV)
       |   |   ||  |
       R4  R5  O   R1
```

dans laquelle $R^1$, $R^3$, $R^4$ et $R^5$ ont les significations ci-dessus, ou on fait réagir un composé répondant à la formule III

```
       *a         *b
R3OOC-CH-N - C -CH-OSO2CF3              (III)
       |   |   ||  |
       R4  R5  O   R1
```

dans laquelle $R^1$, $R^3$, $R^4$ et $R^5$ ont les significations indiquées ci-dessus avec un composé répondant à la formule V

```
           *
R-(CH2)n-CH-NH2                         (V)
           |
           COOR2
```

dans laquelle n, R et $R^2$ ont les significations indiquées ci-dessus, en présence d'au moins un équivalent d'une base qui ne réagit pas avec des composés répondant aux formules II et III, on élimine éventuellement les groupes ester par hydrolyse ou hydrogénolyse et on estérifie éventuellement les groupes carboxy libres d'une manière connue en soi.

2. Procédé selon la revendication 1, caractérisé en ce qu'on prépare des composés de formule I, dans lesquels $R^4$ et $R^5$ forment ensemble avec les atomes de carbone les portant un système éventuellement substitué de la série des: pyrrolidine, pipéridine, tétrahydroisoquinoléine, décahydroisoquinoléine, octahydroindole, octahydrocyclopenta[b]pyrrole, 2-aza-bicyclo[2.2.2]octane, 2-azabicyclo[2.2.1]-heptane, 2-azaspiro[4.5]décane, 2-azaspiro[4.4]-nonane, spiro[(bicyclo[2.2.1]heptane)-2,3-pyrrolidine], spiro[(bicyclo[2.2.2]octane)-2,3-pyrrolidine], 2-azatricyclo[4,3,0,$1^{6,9}$]décane, décahydrocyclohepta[b]pyrrole, octahydroisoindole, octahydrocyclopenta[c]pyrrole, 2,3,3a,4,5,7a-hexahydroindole et tétrahydrothiazole.

3. Procédé selon la revendication 1 ou 2, caractérisé en ce qu'on prépare des composés de formule I, dans lesquels:
n est 1 ou 2,
R représente l'hydrogène,
un groupe alcoyle ayant de 1 à 8 atomes de carbone,
un groupe alcényle ayant de 2 à 6 atomes de carbone,
un groupe cycloalcoyle ayant de 3 à 9 atomes de carbone,
un groupe aryle ayant de 6 à 12 atomes de carbone qui peuvent être mono-, di- ou trisubstitué par un groupe alcoyle en $C_1$-$C_4$, alcoxy en $C_1$-$C_4$, hydroxy, halogéno, nitro, amino, aminoéthyle, alcoyl($C_1$-$C_4$)-amino, di-alcoyl($C_1$-$C_4$)-amino, acyl($C_1$-$C_4$)-amino, de préférence alcanoyl ($C_1$-$C_4$)-amino, méthylènedioxy, carboxy, cyano et/ou sulfamoye,
un groupe alcoxy ayant de 1 à 4 atomes de carbone,
un groupe aryloxy ayant de 6 à 12 atomes de carbone, qui peut être substitué comme décrit pour le groupe aryle,
un groupe hétéroaryloxy mono- ou bicyclique, ayant de 5 à 7 ou de 8 à 10 atomes dans le noyau, dont de 1 à 2 atomes du noyau représentent des atomes de soufre ou d'oxygène et/ou de 1 à 4 atomes du noyau représentent des atomes d'azote, qui peut être substitué comme décrit ci-dessus pour le groupe aryle,
un groupe amino-alcoyle en $C_1$-$C_4$,
un groupe alcanoyl($C_1$-$C_4$)-amino-alcoyle ($C_1$-$C_4$),
un groupe aryol($C_7$-$C_{13}$)-amino-alcoyle ($C_1$-$C_4$),
un groupe alcoxy($C_1$-$C_4$)-carbonylamino-alcoyle ($C_1$-$C_4$),
un groupe aryl($C_6$-$C_{12}$)-alcoxy($C_1$-$C_4$)-carbonyl-amino-alcoyle ($C_1$-$C_4$),
un groupe aryl($C_6$-$C_{12}$)-alcoylamino($C_1$-$C_4$)-alcoyle ($C_1$-$C_4$),
un groupe alcoyl($C_1$-$C_4$)-amino-alcoyle ($C_1$-$C_4$),
un groupe di-alcoyl($C_1$-$C_4$)-amino-alcoyle ($C_1$-$C_4$),
un groupe guanidino-alcoyle ($C_1$-$C_4$),
un groupe imidazoyle, indolyle,
un groupe alcoyl($C_1$-$C_4$)-thio,
un groupe alcoyl($C_1$-$C_4$)-thio-alcoyle ($C_1$-$C_4$),
un groupe aryl($C_6$-$C_{12}$)-thio-alcoyle ($C_1$-$C_4$), qui peut être substitué dans la partie aryle comme décrit ci-dessus pour le groupe aryle,
un groupe aryl($C_6$-$C_{12}$)-alcoyle($C_1$-$C_4$)-thio, qui peut être substitué dans la partie aryle, comme décrit ci-dessus pour le groupe aryle,

un groupe carboxy-alcoyle ($C_1$-$C_4$),
un groupe carboxy, carbamoyle,
un groupe carbamoyl-alcoyle ($C_1$-$C_4$),
un groupe alcoxy($C_1$-$C_4$)-carbamoyl-alcoyle ($C_1$-$C_4$),
un groupe aryloxy($C_6$-$C_{12}$)-alcoyle ($C_1$-$C_4$), qui peut être substitué dans la partie aryle comme décrit ci-dessus pour le groupe aryle, ou
un groupe aryl($C_6$-$C_{12}$)-alcoxy ($C_1$-$C_4$), qui peut être substitué dans la partie aryle comme décrit ci-dessus pour le groupe aryle,

$R^1$ représente l'hydrogène,
un groupe alcoyle ayant de 1 à 6 atomes de carbone,
un groupe alcényle ayant de 2 à 6 atomes de carbone,
un groupe alcynyle ayant de 2 à 6 atomes de carbone,
un groupe cycloalcoyle ayant de 3 à 9 atomes de carbone,
un groupe cycloalcényle ayant de 5 à 9 atomes de carbone,
un groupe cycloalcoyl($C_3$-$C_9$)-alcoyle ($C_1$-$C_4$),
un groupe cycloalcényl($C_5$-$C_9$)-alcoyle ($C_1$-$C_4$),
un groupe aryle éventuellement partiellement hydrogéné, ayant de 6 à 12 atomes de carbone, qui peut être substitué comme décrit pour R ci-dessus,
un groupe aryl($C_6$-$C_{12}$)-alcoyle ($C_1$-$C_4$) ou aroyl($C_7$-$C_{13}$)-alcoyle ($C_1$ ou $C_2$) qui peuvent tous les deux être substitués comme le groupe aryle précédent,
un groupe hétéroaryle mono- ou bicyclique, éventuellement partiellement hydrogéné, ayant de 5 à 7 ou de 8 à 10 atomes dans le noyau, dont de 1 à 2 atomes du noyau représentent des atomes de soufre ou d'oxygène et/ou de 1 à 4 atomes du noyau représentent des atomes d'azote, qui peut être substitué comme le groupe aryle précédent, ou
la chaîne latérale d'un α-amino acide $R^1$-CH-($NH_2$)-COOH d'origine naturelle, éventuellement protégé,

$R^2$ et $R^3$ sont identiques ou différents et représentent l'hydrogène,
un groupe alcoyle ayant de 1 à 6 atomes de carbone,
un groupe alcényle ayant de 2 à 6 atomes de carbone,
un groupe di-alcoyl($C_1$-$C_4$)-amino-alcoyle ($C_1$-$C_4$),
un groupe alcanoyloxy($C_1$-$C_5$)-alcoyle ($C_1$-$C_4$),
un groupe alcoxy($C_1$-$C_6$)-carbonyloxy-alcoyle ($C_1$-$C_4$),
un groupe aroyloxy($C_7$-$C_{13}$)-alcoyle ($C_1$-$C_4$),
un groupe aryloxy($C_6$-$C_{12}$)-carbonyloxy-alcoyle ($C_1$-$C_4$),
un groupe aryle ayant de 6 à 12 atomes de carbone,
un groupe aryl($C_6$-$C_{12}$)-alcoyle ($C_1$-$C_4$),
un groupe cycloalcoyle en $C_3$-$C_9$ ou
un groupe cycloalcoyl($C_3$-$C_9$)-alcoyle ($c_1$-$C_4$),
et

$R^4$ et $R^5$ ont la signification indiquée ci-dessus.

4. Procédé selon l'une quelconque des revendications 1 à 3, caractérisé en ce qu'on prépare des composés de formule I, dans lesquels:

n est 1 ou 2,

R représente un groupoe alcoyle en $C_1$-$C_6$, alcényle en $C_2$-$C_6$, cycloalcoyle en $C_3$-$C_9$, aminoalcoyle en $C_1$-$C_4$, acyl($C_2$-$C_5$)-aminoalcoyle ($C_1$-$C_4$), aroyl($C_7$-$C_{13}$)-aminoalcoyle ($C_1$-$C_4$), alcoxy ($C_1$-$C_4$)-carbonylamino-alcoyle ($C_1$-$C_4$), aryl($C_6$-$C_{12}$)-alcoxy($C_1$-$C_4$)-carbonylamino-alcoyle ($C_1$-$C_4$), aryle en $C_6$-$C_{12}$ qui peut être mono-, di- ou trisubstitué par un groupe alcoyle en $C_1$-$C_4$, alcoxy en $C_1$-$C_4$, hydroxy, halogéno, nitro, amino, alcoyl($C_1$-$C_4$)-amino, di-alcoyl($C_1$-$C_4$)-amino et/ou méthylènedioxy,
ou un groupe 3-indolyle,

$R^1$ représente l'hydrogène ou un groupe alcoyle en $C_1$-$C_6$ qui peut éventuellement être substitué par un groupe amino, acyl($c_1$-$C_6$)-amino ou benzoylamino; un groupe alcényle en $C_2$-$c_6$), cycloalcoyle en $C_3$-$C_9$, cycloalcényle en $C_5$-$C_9$, cycloalcoyl($C_3$-$C_7$)-alcoyle ($C_1$-$C_4$), aryle en $C_6$-$C_{12}$ , ou aryle partiellement hydrogéné, qui peuvent chacun être substitués par un groupe alcoyle en $C_1$-$C_4$, alcoxy en $C_1$ ou $C_2$ ou par un halogène; un groupe aryl($C_6$-$C_{12}$)-alcoyle ($C_1$-$C_4$) ou aroyl($C_7$-$C_{13}$)-alcoyle ($C_1$-$C_2$), qui peuvent être tous les deux substitués comme défini ci-dessus pour le groupe aryle; un groupe hétérocyclique mono- ou bicyclique ayant de 5 à 7 ou de 8 à 10 atomes dans le noyau, dont de 1 à 2 atomes du noyau sont des atomes de soufre ou d'oxygène et/ou de 1 à 4 atomes du noyau sont des atomes d'azote; ou une chaîne latérale d'un α-amino acide d'origine naturelle, éventuellement protégé,

$R^2$ et $R^3$ sont identiques ou différents et représentent l'hydrogène, un groupe alcoyle en $C_1$-$C_6$, alcényle en $C_2$-$C_6$ ou aryl($C_6$-$C_{12}$)-alcoyle ($C_1$-$C_4$) et

$R^4$ et $R^5$ ont la signification indiquée ci-dessus.

5. Procédé selon l'une quelconque des revendications 1 à 4, caractérisé en ce qu'on prépare des composés de formule I, dans lesquels:

n est 1 ou 2,

R représente un groupe méthyle, éthyle, cyclohexyle, tert-butoxy-carbonylamino-alcoyle ($C_1$-$C_4$) ou phényle, qui peut être mono- ou disubstitué par un groupe phényle, alcoyle en $C_1$ à $C_2$, alcoxy en $C_1$ ou $C_2$, hydroxy, fluoro, bromo, chloro, amino, alcoylamino en $C_1$-$C_4$, di-alcoyl($C_1$-$C_4$)-amino, nitro et/ou méthylènedioxy, ou peut être trisubstitué dans le cas d'un groupe méthoxy,

$R^1$ représente l'hydrogène, un groupe alcoyle en $C_1$-$C_3$, alcényle en $C_2$ ou $C_3$, la chaîne latérale éventuellement protégée de la lysine, un groupe benzyle, 4-méthoxybenzyle, 4-éthoxybenzyle, phénéthyle, 4-aminobutyle ou benzoylméthyle,

$R^2$ et $R^3$ sont identiques ou différents et représentent l'hydrogène, un groupe alcoyle en $C_1$-$C_4$ ou benzyle, et

$R^4$ et $R^5$ ont la signification indiquée ci-dessus.

6. Procédé de préparation de composés de formule III, dans laquelle:

6. Procédé de préparation de composés de formule III, dans laquelle:

$R^1$ représente l'hydrogène ou un groupe alcoyle en $C_1$-$C_6$, qui peut être éventuellement substitué par un groupe amino, acyl-($C_1$-$C_6$)amino ou benzoylamino; un groupe alcényle en $C_2$-$C_6$, cycloalcoyle en $C_3$-$C_9$, cycloalcényle en $C_5$-$C_9$, cycloalcoyl-($C_3$-$C_7$)-alcoyle ($C_1$-$C_4$), aryle en $C_6$-$C_{12}$ ou aryle partiellement hydrogéné, qui peuvent chacun être substitués par un groupe alcoyle en $C_1$-$C_4$, alcoxy en $C_1$ ou $C_2$ ou par un halogène; un groupe aryl($C_6$-$C_{12}$ )-alcoyle ($C_1$-$C_4$) ou aroyl($C_7$-$C_{13}$)-alcoyle ($C_1$-$C_2$) qui peuvent être tous les deux substitués dans la partie aryle comme défini précédemment; un groupe hétérocyclique mono- ou bicyclique ayant de 5 à 7 ou de 8 à 10 atomes dans le noyau, dont de 1 à 2 atomes du noyau sont des atomes de soufre ou d'oxygène et/ou de 1 à 4 atomes du noyau sont des atomes d'azote, ou une chaîne latérale d'un α-amino acide d'origine naturelle, éventuellement protégé,

$R^3$ représente l'hydrogène, un groupe alcoyle en $C_1$-$C_6$, alcényle en $C_2$-$C_6$ ou aryl($C_6$-$C_{12}$ )-alcoyle ($C_1$-$C_4$) et

$R^4$ et $R^5$ ont la signification indiquée pour les composés de formule I,

caractérisé en ce qu'on fait réagir des dérivés d'acides α-hydroxycarboxyliques de formule VII

$$R^3OOC\text{-}\overset{*a}{\underset{R^4}{CH}}\text{-}\underset{R^5}{N}\text{-}\underset{O}{C}\text{-}\overset{*b}{\underset{R^1}{CH}}\text{-}OH \qquad (VII)$$

dans laquelle $R^1$, $R^3$, $R^4$ et $R^5$ ont les significations indiquées ci-dessus, avec un agent de trifluorométhanesulfonation, éventuellement en présence d'une base.

7. Procédé selon la revendication 6, caractérisé en ce que

$R^1$ représente l'hydrogène, un groupe alcoyle en $C_1$-$C_3$ ou alcényle en $C_2$ à $C_3$, la chaîne latérale eventuellement protégée de la lysine, un groupe benzyle, 4-méthoxybenzyle, 4-éthoxybenzyle, phénéthyle, 4-aminobutyle ou benzoylméthyle,

$R^3$ représente l'hydrogène, un groupe alcoyle en $C_1$-$C_4$ ou benzyle, et

$R^4$ et $R^5$ ont la signification indiquée pour les composés de formule I.

8. Procédé selon la revendication 6, caractérisé en ce que

$R^1$ représente un groupe méthyle, éthyle, phényle, la chaîne latérale éventuellement acylée de la lysine, un groupe benzyle ou la chaîne latérale O-alcoylée en $C_1$-$C_6$ de la tyrosine,

$R^3$ représente l'hydrogène, un groupe méthyle, éthyle, tert-butyle ou benzyle et

$R^4$ et $R^5$ ont la signification indiquée pour les composés de formule I.